(19)

Europäisches Patentamt

European Patent Office

Office européen des brevets

(11) **EP 3 639 743 A1**

(12) **EUROPEAN PATENT APPLICATION**

(43) Date of publication:
**22.04.2020 Bulletin 2020/17**

(51) Int Cl.:
***A61B 5/145*** (2006.01)   ***A61B 5/00*** (2006.01)

(21) Application number: **18200903.5**

(22) Date of filing: **17.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PELSSERS, Eduard Gerard Marie**
**5656 AE Eindhoven (NL)**

• **JOHNSON, Mark Thomas**
**5656 AE Eindhoven (NL)**
• **VAN LIESHOUT, Ron Martinus Laurentius**
**5656 AE Eindhoven (NL)**
• **GERHARDT, Lutz Christian**
**5656 AE Eindhoven (NL)**
• **DELLIMORE, Kiran Hamilton J.**
**5656 AE Eindhoven (NL)**
• **MARTEIJN, Ruben**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **SYSTEM AND METHOD FOR DETERMINING A SWEAT EXCRETION RATE OF A USER**

(57)     According to an aspect, there is provided a system (1) for determining a sweat excretion rate of a user. The system (1) comprises: a fluidic structure (2) configured to be in contact with the skin of the user to collect sweat excreted at the surface of the skin of the user from sweat glands; and a processor (3). The fluidic structure (2) comprises: a plurality of sample areas (4), each sample area (4) comprising a cavity (5) configured to gather excreted sweat, and two or more of the plurality of sample areas (4) comprising a sensor (6) configured to acquire sensor data (7) relating to an excretion rate of the excreted sweat at the sample area (4). The processor (3) is configured to: receive the sensor data (7) from two or more of the sensors (6) of the fluidic structure (2); determine, from the received sensor data (7), the number of sweat glands at each sample area (4) and a total sweat excretion rate of the sample areas (4); determine a total number of sweat glands from the number of sweat glands at each sample area (4); and determine a sweat excretion rate per sweat gland using the total sweat excretion rate and the total number of sweat glands. According to another aspect, there is provided a method for determining a sweat excretion rate of a user.

Figure 1

**Description**

FIELD OF THE INVENTION

Embodiments of the present invention relate generally to determining the sweat excretion rate of a user.

BACKGROUND OF THE INVENTION

Non-invasive, continuous and prolonged monitoring of biomarkers that indicate health and well-being is in demand. For example, for monitoring dehydration, stress, sleep, children's health and in perioperative monitoring. Sweat is a non-obtrusively accessible bio-fluid containing physiologically and metabolically rich information. The development of reliable sweat sensing has been hampered by several issues: (1) results from sweat sensing have been highly variable; (2) a correlation between blood and sweat values appears to be lacking for various biomarkers; (3) the focus of sweat sensing has been on sensors, not on reliable and robust collection methods for the minute amounts produced. Some examples of clinically relevant components of sweat are: Na+, Cl- and/or K+ to monitor dehydration; Lactate as an early warning for inflammation; glucose for diabetics & neonates; and cortisol for sleep and stress monitoring.

Table 1 shows a non-exhaustive list of biomarkers known to be present in sweat, underlining the promise and relevance of sweat sensing to a diversity of applications. In fact, the concept of using sweat for non-invasive access to biomarkers and solutes in blood is not new, with clinical work showing promising results as far back as the 1940s and 1950s. However, to date, the impactful application of sweat analysis has been limited mainly to cystic fibrosis (CF) diagnostics, and testing for abuse of drugs and alcohol. However, there are known limitations of using sweat as a clinical sample, such as the difficulty to produce enough sweat for analysis, sample evaporation, lack of appropriate sampling devices, need for trained staff and normalisation of sampled volume. Furthermore, the correlation between blood and sweat values appears to be lacking for various biomarkers and the results from sweat sensing have been highly variable.

*Table 1: Examples of relevant biomarkers in sweat (LOD=required limit of detection)*

| Marker | LOD | Applications | Status |
|---|---|---|---|
| Na+, K+, Cl- | 10 mM | Dehydration monitoring; Cystic Fibrosis diagnosis; | CF diagnosis from sweat in NICU clinical practice |
| Lactate | 10 mM | Pressure ischemia detection; Post-operative monitoring | Predictive ischemia marker shown; Only temporal correlation with blood shown |
| Urea | 1 mM | Skin conditions; Dehydration monitoring | Hypothesis: urea content in sweat keeps skin in good condition |
| Ethanol | 1 mM | Alcohol use monitoring | On-skin monitoring used in practice |
| Ammonium | 1 mM | Metabolic disorders | Absolute correlation to blood shown |
| Glucose | 0.1 mM | Diabetes | Temporal correlation to blood shown |
| Cortisol | $10^{-4}$ mM | Stress monitoring | Absolute correlation to blood shown |

In part, these issues are now being resolved by bringing wearable sensors into almost immediate contact with sweat as it emerges from the skin. However, the focus of such systems studies is generally on the development and the integration of the sensors themselves, rather than the sweat sample collection. Sweat samples are mostly collected by placing several $cm^2$ sized absorbent pads between the skin and the sensor. The assumption is that, provided that ample sweat is produced (hence tests are always done on individuals that are heavily exercising), the pad will absorb the sweat for analysis, and newly generated sweat will refill the pad and 'rinse away' the old sweat. However, it is likely that the time-dependent response of the sensor does not directly reflect the actual level of biomarkers over time due to accumulation effects. Accordingly, known approaches to sweat sample collection and presentation to the sensors are not well controlled, making continuous reliable sensing over a long period of time difficult, and the patches are not designed to handle the tiny amounts of sweat that are produced under normal conditions, i.e. in the order of sub-nano to nanolitres per minute, per sweat gland.

Improving the unintended variability in measuring biomarker concentration in sweat is therefore a topic of interest. Eccrine sweat glands have a mechanism to secrete plasma like fluid into the tubes of the gland's coiled portion. However, when transported through the dermal duct a partial re-absorption of components takes place. Additionally, particular ions are re-absorbed and consequently, while the plasma-like fluid is close to the osmotic value of blood, the sweat exiting the duct is less than osmotic. It has been measured that the concentration of certain components in sweat increases with an increasing level of sweat production. This is caused by the fact that the re-absorption of components cannot proportionally follow the increase in secretion rate.

It is noted that, in this specification, the word 'secretion' is reserved for the plasma like fluid being ejected into the coiled tubular portion of the sweat gland and the word 'excretion' is reserved for the sweat exiting the duct of a sweat gland onto the skin.

In summary, the concentration of particular biomarkers in excreted sweat varies as a function of the excretion rate per gland. To attribute a concentration of a particular biomarker to a disorder, it is necessary to know at which excretion rate the concentration is measured, and only then may it be determined whether a concentration is deviating from the normal value.

The total excretion rate of a particular sampled skin area depends on two parameters: (i) the excretion rate per gland; and (ii) the number of active glands in the sampled skin area. Unfortunately, whilst the secretion rate per gland is rather uniform (local gland to gland variation estimated typically +/-10%), the number of active glands does vary considerably and consequently the desired excretion rate per gland cannot be determined from the total excretion rate of the sampled skin area. Therefore, the concentration of a particular sweat component may point to a (clinical) disorder but may also be attributed to an increase in sweat rate. This is a serious problem for sweat monitoring devices. Means of determining the total sweat excretion rate are known in the art, yet, considering the above, this is an ambiguous parameter because the number of sweat glands is unknown.

The typical number of glands per surface area is known for various body locations (back, axilla, etc.). However, there is a large individual variation on this typical number and so it is not possible to determine the actual number of glands per surface area using the typical values alone. This is especially true when a small sampling area is being sampled by a device. Laboratory methods associated with determining the number of glands per surface area are known, but these require time intensive laborious work and staining agents. Such techniques are therefore impractical and unfavourable for routine measurements.

SUMMARY OF THE INVENTION

According to an embodiment of a first aspect, there is provided a system for determining a sweat excretion rate per sweat gland of a user. The system comprises: a processor; and a fluidic structure configured to be in contact with the skin of the user to collect sweat excreted at the surface of the skin of the user from sweat glands. The fluidic structure comprises: a plurality of sample areas, each sample area comprising a cavity configured to gather excreted sweat, and two or more of the plurality of sample areas comprising a sensor configured to acquire sensor data relating to an excretion rate of the excreted sweat at the sample area. The processor is configured to: receive the sensor data from two or more of the sensors of the fluidic structure; determine, from the received sensor data, the number of sweat glands at each sample area and a total sweat excretion rate of the sample areas; determine a total number of sweat glands from the number of sweat glands at each sample area; and determine a sweat excretion rate per sweat gland using the total sweat excretion rate and the total number of sweat glands.

Thus, using sensor data acquired from sensors in the fluidic structure and relating to an excretion rate of the excreted sweat at the sample areas, the system is able to determine the number of sweat glands at a sampled skin area and the sweat excretion rate per sweat gland. The sweat excretion rate per sweat gland is an average sweat excretion rate per sweat gland. Specifically, the sensor data is received and processed by the processor to determine the number of sweat glands at each sample area, a total sweat excretion rate of the sample areas, a total number of sweat glands and an average sweat excretion rate per sweat gland.

Embodiments of the present invention may be considered to provide a fluidic structure, which may, for example, be provided in a patch like format, in concert with a processor that enables determination of the sweat excretion rate per gland of a particular sampled skin surface area, which may be achieved, for example, by implementing a smart algorithm. The algorithm may, for example, be Poisson distribution based. That is, embodiments of the invention provide a system which combines a means for collecting data on excreted sweat and for processing the data to determine the average sweat excretion rate per sweat gland.

In the fluidic structure, the excreted sweat at a sample area interacts with a sensor so that the sensor acquires the sensor data. A cavity is provided at each sample area to gather the excreted sweat. Specifically, the fluidic structure is arranged such that, when the structure is in contact with the skin, the cavity is placed over a portion of skin so that the excreted sweat is collected within the cavity and the cavity is to be filled by the excreted sweat. The size of the cavity determines the amount of sweat required to fill the cavity and the area of the skin that is covered by the cavity. The cavities of the structure may all be the same size or they may be of different sizes. The sensor of a given sample area may be disposed in the cavity or the sensor may be disposed separately from the cavity in the sample area.

The sensor may, for example, be a flow sensor that measures a flow rate of the excreted sweat at the sample area. The acquired sensor data indicates the excretion rate of the sweat at the sample area at which the sensor is disposed. The excretion rate may also be referred to as a flow rate or a sweat rate. A sensor may be provided at each sample area of the fluidic structure such that the number of sensors is equal to the number of sample areas. Conversely, some sample areas may not comprise a sensor so that there are more sample areas than sensors in the fluidic structure. The fluidic structure may also comprise different types of sensors such that the same type of sensor is not provided at each sample area.

The fluidic structure may be provided as a patch that is arranged to be positioned at certain location of the body to collect and process sweat. That is, the fluidic structure may be attached to or connected in direct contact with the skin of the user at a given location so as to directly gather the sweat excreted by sweat glands at the skin. The fluidic structure may comprise adhesive for attaching the structure to the skin of the user. For example, adhesive may be provided at the outer edge of the fluidic structure, at the outer edge of one or more cavities and/or on the portion of the fluidic structure between cavities. The adhesive may be arranged such that it does not prevent excreted sweat from being collected in the cavities of the structure. In some embodiments, the adhesive may be arranged between the cavities such that excreted sweat cannot pass between the cavities. However, in some embodiments, the adhesive may be arranged to allow sweat to pass between adjacent cavities.

The processor (data processing unit) receives, from the fluidic structure, the data acquired from the sensors. The sensor data relates to an excretion rate at the sample area at which the sweat was collected and so the processor is able to determine, from the sensor data, the excretion rate at the sample areas corresponding to the received sensor data. The processor is also able to determine the total sweat excretion rate at the sample areas. Furthermore, by processing the received data, the processor determines the number of sweat glands at each sample area and the average sweat excretion rate per sweat gland. The determined number of sweat glands at a given sample area will, in dominance, be a whole number but, in minority, a sweat gland may be partially covered at the border of a sample area. The sweat excretion rate at the sample area may not be a whole number and may, for example, be a number to two decimal places. The processing performed by the processor may, for example, include comparison and discretization of the received sensor data.

The user may also be referred to as a patient, human subject, individual, person of interest, etc. and may be an individual whose sweat is being monitored to assess their health and well-being. Sweat glands are arranged in the skin such that they excrete sweat out of the surface of the skin. Glands that excrete sweat may be considered to be active glands, such that if an excretion rate at a sample area is at or close to zero, it is considered that there are no glands or, more specifically, no active glands at the sample area. Gland activity may change over time.

As discussed above, according to embodiments of the present invention, the sensor data acquired at multiple sample areas is used to determine the number of sweat glands at the sample areas and the average sweat excretion rate per sweat gland at the sampled skin area at which the fluidic structure is positioned. Since the sweat excretion rate per sweat gland is determined, further analysis of the excreted sweat may be accurately performed. For example, the determination of biomarkers in excreted sweat that are dependent on and vary as a function of the excretion rate per gland may be accurately assessed and analysed. It is therefore possible to tie measured properties of the excreted sweat to the determined sweat excretion rate per sweat gland so that evaluation of the properties may be correctly performed. It is also possible to distinguish between a (clinical) disorder indicated by, for example, a concentration of a particular parameter in excreted sweat, and an increase in sweat rate, for example, due to multiple sweat glands, which results in a similar concentration of the parameter. Determination of the number of sweat glands per sample area is therefore beneficial.

The arrangement of the sample areas and thus the cavities in the fluidic structure may be determined by the application and/or may be determined by the type of sensor used in the sample areas. For example, the cavities may be arranged directly adjacent to each other or the cavities may be arranged with a small distance between the cavities. If the cavities are arranged directly adjacent to each other, then the sweat gathered in a first cavity may be able to flow into an adjacent cavity when the first cavity is full. If the cavities are spaced apart, then the spacing between two cavities may be at least equal to the size of a typical sweat gland so as to prevent excreted sweat from one sweat gland being gathered in two (or more) cavities and thus spread over two (or more) sample areas.

According to a preferred embodiment, the sensor data may be an excretion rate measured at the sample area. The number of sweat glands at each sample area may be determined by: determining, from the received sensor data, a zero excretion rate that corresponds to zero sweat glands at a sample area such that sensor data below a predetermined threshold is categorised as zero excretion rate; determining, from the received sensor data, a base excretion rate that corresponds to one sweat gland at a sample area such that multiples of the base excretion rate correspond to multiples of one sweat gland; and categorising the sensor data from each sample area according to the zero excretion rate, the base excretion rate and multiples of the base excretion rate to determine the number of glands at each sample area.

The sensors of the fluidic structure may therefore measure an excretion rate at the respective sample areas and the measured excretion rates may then be used to determine a base rate and categorise the measurements in accordance with the base rate. More specifically, the measured excretion rates for the sample areas may be compared and discretized to determine the number of sweat glands at a sample area. The excretion rates at the sample areas may be measured with respect to time, such that the excretion rates of the sample areas are all measured at the same point in time and the comparison between measured excretion rates reflects the differences between the excretion rates at the certain point in time. The units of the measured excretion rate may be determined by the size of the fluidic structure and/or the size of the cavities. For example, the measured excretion rates may be in units of nanolitres per minute (nl/min).

The measured excretion rates may be compared to determine the base excretion rate (base rate) and multiples of the base excretion rate (multiple rate). The base excretion rate may be seen as a common factor or largest common divisor of all of the measured excretion rates such that the base excretion rate divides the multiples of the base excretion rate, i.e. the multiples of the base excretion rate may be divided by the base excretion rate such that the multiples are whole numbers of one or greater. The measured excretion rates may be rounded to an appropriate number of decimal places (for example, two decimal places) and/or the comparison may be approximated, such that the base excretion rate is approximately the largest common divisor of the measured excretion rates and the multiples of the base excretion rate are not exact multiples but approximate whole numbers of one or more. A detailed example of measured excretion rates and the determination of the base excretion rate and multiples of the base excretion rate is provided in the detailed description.

The zero excretion rate (zero rate) may be determined as the measured excretion rates that are equal to zero or close to zero, i.e. below a predetermined value or threshold, such as, for example, below 0.02 nl/min/gland. The threshold may, for example, be determined by the average excretion rate of persons in relative rest, which has been shown to be approximately 0.1 nl/min/gland for females and approximately 0.3 nl/min/gland for males. It may therefore be considered that no sweat is excreted at the sample areas with such low measured excretion rates and that there are no active sweat glands located at the sample area. Such low, negligible measurements may be the result of, for example, noise or interference detected by the sensor.

By categorising the sample areas in accordance with the zero excretion rate, the base excretion rate and multiples of the base excretion rate, it may be possible to determine the number of sweat glands at each sample area. Specifically, if a sample area is categorised as zero excretion rate, then it may be determined that there are zero sweat glands at that sample area. Similarly, if the sample area is categorised as the base excretion rate, then it may be determined that there is a single sweat gland at the sample area and categorisation as multiples of the base excretion rate results in a determination of multiple sweat glands at the sample area, for example, two, three, etc., sweat glands.

When the measured excretion rates are categorised as discussed above, the processor may additionally: determine the total sweat excretion rate of the sample areas by summing the sensor data from all sample areas other than sample areas categorised as zero excretion rate; determine the total number of sweat glands by summing the determined number of glands at each sample area; and determine the sweat excretion rate per sweat gland by dividing the total sweat excretion rate by the total number of sweat glands. Thus the calculated sweat excretion rate per sweat gland may be an average sweat excretion rate per sweat gland.

Thus, from the categorisation of the sample areas as zero rate, base rate and multiple rate, the total number of sweat glands may be determined by adding the number of sweat glands at each sample area, indicated by the categorisation. The total excretion rate of the sampled area of skin, corresponding to the positioning and surface area covered by the fluidic structure, may also be determined by adding the measured excretion rates of the sample areas. Any measurements acquired at sample areas categorised as the zero excretion rate (for example, negligible measurements at or close to zero) may be ignored and not used when determining the total excretion rate. Finally, the total sweat excretion rate may be divided by the total number of sweat glands to determine an average sweat excretion rate per sweat gland. The measured excretion rates at each sample area may therefore be used to determine the number of sweat glands at each sample area, the total number of sweat glands covered by the fluidic structure, the total excretion rate of the sweat glands covered by the fluidic structure and the average excretion rate of a single sweat gland.

At least one of the size of each of the cavities and the number of cavities of the fluidic structure may be determined by one or more of the following: the location at which the fluidic structure is in contact with the skin of the user; the predicted number of sweat glands at the location; and the probability that there are zero sweat glands in a sample area at the location. That is, the size and/or number of cavities may be determined by the position at which the fluidic structure is in contact with the skin of the user and/or the expected properties of the skin at that position. For example, different locations on the user's body may have different numbers of expected sweat glands. Some body areas may be expected to have more sweat glands than other areas and so the probability of having zero sweat glands at a sample area also varies in accordance with the predicted number of sweat glands and the size of the sweat glands.

The number and/or size of the cavities may be set so that the chance of there being zero sweat glands at the location of the fluidic structure is minimised. Additionally or alternatively, the size of the cavities may be determined based on the amount of sweat that is expected to be excreted at the location on the user at which the fluidic structure is positioned. For example, if there is expected to be a large amount of sweat, then the cavities may be larger than a location at which there is expected to be a small amount of sweat excretion. The area of the fluidic structure covering the skin of the user may be the total surface area size of all of the sample areas plus the area of the borders and spaces between the sample areas, which may be equal to two thirds of the total surface area size of all of the sample areas. That is, the sample areas may form a third of the total surface area of the structure, with the remaining two thirds of the area being formed from borders and spaces between the sample areas.

Since each sample area comprises a cavity, it may also be seen that the number of sample areas (sample chambers) and the size of the sample areas in the fluidic structure may be determined by the specific application of the system. For example, the number of sample areas and/or size of the sample areas may be determined by the location of the fluidic structure on the user, the expected number of sweat glands at the location, considerations of the user, for example, activity level, age, etc., and/or property of the excreted sweat to be monitored. Dependent on the above listed factors, embodiments of the present invention may provide, for example, a fluidic structure comprising 10, 13, 17 or 96 sample areas and thus 10, 13, 17 or 96 cavities, respectively. An exemplary size of a cavity according to embodiments of the present invention may have diameter of $360\mu$m and a $50\mu$m height. Conversely, the cavity may, for example, have a diameter of 1130 $\mu$m, and a height of $50\mu$m. Larger cavities (cavities with larger volumes) are more likely to cover a higher number of sweat glands and so it may be seen that the probable number of sweat glands covered by each cavity increases with the size of the cavities. That is, a larger cavity may be more likely to cover more sweat glands. The size of the sample areas and the cavities may therefore appropriately match the expected level of sweat excretion at the position at which the fluidic structure is disposed.

It may be preferable for one or more of the plurality of cavities to be segregated into a plurality of conical structures. The centre of each conical structure may be in contact with the skin when the skin is in a first position and not in contact with the skin when the skin is in a second position such that excreted sweat flows between the conical structures. Due to elasticity of the skin, the skin may move between different positions as the user moves. Thus, portions of the fluidic structure which may be in contact with the skin at one position may not be in contact with the skin at another position. In other words, cavities of the fluidic structure may be segregated into conical structures, with the central point of each of these structures initially in contact with the skin of the user. When the user moves, one or more of the central points of the conical structures may no longer be in contact with the skin such that sweat gathered in one conical structure may move to another conical structure.

This may be particularly beneficial for cavities which cover a large surface area of the skin. The elasticity of the skin may cause movement, towards the sensor, of the sweat gathered in such cavities to be impeded or blocked unless the height of the cavity is sufficiently high. However, this will lead to an increase in the volume of the cavity and an increase in the time taken to fill the cavity. The conical structures may therefore ensure that the volume of the cavity is limited without movement of the sweat towards the sensor being blocked. Thus, the conical structures allow for the cavity to cover a larger area of skin without substantially increasing the volume of the cavity or delaying the time taken for the sweat to interact with the sensor.

At least part of the fluidic structure may be composed of one of: a hydrophobic material; and a hydrophilic material. The fluidic structure may therefore be entirely or partially made of a hydrophobic material; entirely or partially made of a hydrophilic material; or made of a mix of hydrophilic and hydrophobic materials. Different parts of the structure may be made of different materials depending on their function and/or the application of the fluidic structure. For example, the areas of the fluidic structure between sample areas and cavities may be composed of a hydrophobic material so that these areas are resistant to excreted sweat. That is, the cavities may be made of either hydrophilic or hydrophobic materials and the type of material may be determined by the application of the structure, such as, for example, the size of the cavities and the positioning of the structure on the user.

The system may further comprise a concentration sensor configured to determine a concentration of a compound in the excreted sweat. The concentration sensor may be disposed in a sample area of the structure, such as in the cavity, and/or may be disposed close to the position of the sensor in the sample area. There may also be multiple concentration sensors such that each concentration sensor is associated with one or more of the cavities. There may also be multiple concentration sensors with each sensor determining a concentration of a different compound. Embodiments of the present invention may therefore also provide means for determining the concentration of compounds in the excreted sweat. This may be combined with the determination of the excretion rate per sweat gland to provide a concentration of a biomarker as a function of the excretion rate per gland. Accurate determination and diagnosis of conditions indicated by biomarkers in the sweat may therefore be achieved.

Embodiments of the system may comprise three or more triangularly arranged sample areas. The processor may be configured to: determine, from the received sensor data, the excretion rate at each of the three sample areas at a first time point; determine, from the received sensor data, the excretion rate at each of the three sample areas at a second time point occurring after the first time point; calculate a first time point ratio indicating a ratio of the excretion rates at the three sample areas at the first time point; calculate a second time point ratio indicating a ratio of the excretion rates at the three sample areas at the second time point; calculate a difference between the first time point ratio and the second time point ratio; and determine, in response to the difference between the first time point ratio and the second time point ratio exceeding a predetermined threshold, degradation of the fluidic structure. Each of the triangularly arranged sample areas may comprise a sensor such that the received sensor data includes excretion rates of the sample areas.

By comparing the ratios of the excretion rates between adjacent sample areas over time, degradation of the fluidic structure may be assessed to verify that the acquired sensor data is accurate. By determining the ratio between the triangularly arranged sample areas, it is possible differentiate between a change in a detected excretion rate due to reduced sweat excretion (for example, due to change in user activity and/or environment) and a change in the detected excretion rate due to degradation of the fluidic structure. That is, it is possible to distinguish between an accurately measured change of the detected excretion rate and an erroneous change in detected excretion rate. This is because the ratios of the sample areas reflect a change of an excretion rate at one sample area with respect to another and so comparison of ratios over time will demonstrate whether the change is consistent over the sample areas. If the change is not consistent, then this may indicate errors in the detected excretion rates and thus degradation of the structure.

The processor may further generate an alarm or notification if degradation of the device is detected. For example, if it is determined that the structure has degraded, then an alarm or notification may be generated to indicate that the structure should be replaced, repaired, recalibrated, etc.

The system may further comprise a supplementary fluidic structure configured to be in contact with the skin of the user to collect sweat excreted at the surface of the skin of the user from sweat glands. The supplementary fluidic structure may comprise: a supplementary sample area comprising a supplementary cavity configured to gather excreted sweat; a supplementary channel connected to the supplementary cavity; and a concentration sensor disposed in the supplementary channel and configured to determine a concentration of a compound in the excreted sweat. The supplementary sample area may be arranged such that the excreted sweat at the supplementary sample area at least partially fills the supplementary cavity, flows into and along the supplementary channel and interacts with the concentration sensor. The size of the supplementary cavity of the supplementary fluidic structure may be at least one hundred times larger than a cavity of the plurality of cavities of the fluidic structure. The surface area of the supplementary cavity covering the skin of the user may, for example, be 1cm$^2$. The supplementary cavity may have a height of 50μm.

The supplementary fluidic structure may therefore gather sweat for the purpose of determining a concentration of a compound in the excreted sweat. The supplementary fluidic structure may be provided in cooperation with the fluidic structure (which may also be referred to as a primary fluidic structure) such that concentration may be determined with respect to the excretion rate per sweat gland. The large surface area covered by the supplementary fluidic structure compared to the primary fluidic structure means that the supplementary fluidic structure is likely to cover a large number of sweat glands, so that the flow rate at the supplementary fluidic structure will be larger and there is no delay in the excreted sweat interacting with the sensor. With no delay in the sweat interacting with the sensor, the concentration measured at a given time point may be accurately correlated with the excretion rate per sweat gland for the given time. The supplementary fluidic structure may be positioned in contact with the skin of the user at a location in close proximity to the primary fluidic structure. The supplementary fluidic structure may also be provided as part of the primary fluidic structure, such that the primary fluidic structure is effectively segregated into two parts.

According to a preferred embodiment one or more of the plurality of sample areas may comprise a channel connected to the cavity. The sensor may be a flow sensor disposed in the channel and may measure an excretion rate of excreted sweat at the sample area. The channel and the sample area may be arranged such that the excreted sweat at the sample area at least partially fills the cavity, flows into and along the channel and interacts with the flow sensor.

The channel may therefore effectively provide an outlet for the cavity, such that the sweat gathered in the cavity of the sweat flows out of the cavity through the channel. The sensor may be disposed in the channel. The channel may be centrally located at the top of the cavity, i.e. the top surface of the cavity which faces the surface of the skin. The channel may comprise a cylindrical channel attached to the main cavity and a rectangular channel attached to the cylindrical channel. One or more concentration sensors may be disposed in the channel.

The flow sensor may comprise an upstream temperature sensor; a downstream temperature sensor; and a pulsed heating element. The excretion rate may be measured by: calculating the difference between a temperature measured by the upstream temperature sensor and a temperature measured by the downstream temperature sensor with respect to the pulsed heating element to derive a flow velocity measurement; and by integrating the flow velocity measurement as a function of time to obtain the excretion rate. The pulsed heating element may be disposed between the upstream temperature sensor and the downstream temperature sensor. The sensor may be pre-calibrated in laboratory testing using different fluids.

The fluidic structure may comprise a primary intersection and an exit channel connected to the primary intersection and configured to remove the excreted sweat from the fluidic structure. One or more of the plurality of channels may be connected to the primary intersection such that the excreted sweat flows through the channel, into the primary intersection and then into the exit channel. One or more concentration sensors may be disposed in the exit channel.

The channels are therefore combined at the primary intersection and connected to the exit channel such that the sweat exits the structure. The system may further comprise means for removing the sweat from the exit channel. For example, an absorber pad may be attached to the exit channel (at the end which is not attached to the primary intersection) to remove the excreted sweat.

Furthermore, the structure may comprise a secondary intersection disposed between two or more of the channels and the primary intersection. The two or more channels may be connected to the secondary intersection so as to combine the channels prior to connection with the primary intersection. That is, a channel, comprising the combined two or more channels may connect the secondary intersection to the primary intersection. One or more concentration sensors may be disposed between the primary intersection and the secondary intersection. The structure may comprise multiple secondary intersections, each combining two or more channels between the sample areas and the primary intersection. If there are multiple secondary intersections, then some or all of the combined channels may comprise one or more concentration sensors between the secondary intersection and the primary intersection.

The structure may further comprise: a fluid leveller disposed in one or more of the plurality of cavities and composed of a hydrophilic material. The fluid leveller may be configured to direct the excreted sweat to the channel. The fluid leveller may be considered as a hydrophilic membrane that is disposed across the width of the cavity such that the surface of the leveller faces a surface of the skin, i.e. in a direction parallel with the surface of the skin. The fluid leveller may attract the excreted sweat such that the sweat hits the membrane and flows into the upper part of the cavity, towards the channel. That is, the arrangement of the fluid leveller defines a lower part of the cavity, adjacent to the skin, and an upper part, adjacent to the channel. The sweat may hit the hydrophilic membrane and flow over the membrane into the upper part and so it is not necessary for the cavity to completely fill up before the sweat flows into the channel.

The fluid leveller may be disposed in cavities that are made of either a hydrophilic material or a hydrophobic material. The fluid leveller may preferably be disposed in a cavity with a large volume. The fluidic structure may comprise a plurality of fluid levellers such that a fluid leveller is disposed in each of one or more of the cavities.

The structure may further comprise a de-bubbler configured to eliminate air bubbles formed in the excreted sweat. The de-bubbler may comprise: a first part comprising a plurality of hydrophilic projections arranged sequentially to define a plurality of channels between the hydrophilic projections; and a second part comprising a hydrophobic material and a vent hole. The first part may be disposed opposite the second part; and the de-bubbler may be disposed in one or more of: a channel, and the exit channel. The de-bubbler may further comprise a hydrophobic membrane disposed across (covering) the vent hole. Air bubbles may form in the system due to the flow of the sweat. Air bubbles may be problematic for some types of sensors, which may prevent sensor data being acquired or result in inaccurate sensor data. It may therefore beneficial to remove these air bubbles from the sweat.

According to an alternative preferred embodiment of the invention, the sample areas of the plurality may be arranged adjacent to each other such that excreted sweat collected in a first cavity is able to overflow into an adjacent cavity. The sensor may be a colouration sensor disposed in a cavity of the plurality and configured to activate a colour change of the sample area in response to interaction with excreted sweat. The sensor data may be data indicating a colouration of a sample area. The processor may be configured to determine the number of sweat glands by: determining, from the received sensor data, a zero excretion rate that corresponds to zero sweat glands at a sample area at which there is no colouration; determining, from the received sensor data, a base excretion rate that corresponds to one sweat gland at a sample area at which there is colouration at a single sample area; determining, from the received sensor data, multiples of the base excretion rate that corresponds to multiples of one sweat gland at a sample area at which there is colouration at multiple adjacent sample areas; and categorising the sensor data from each sample area according to the zero excretion rate, the base excretion rate and multiples of the base excretion rate to determine the number of glands at each sample area.

The colourisation of the cavities resulting from the excreted sweat interacting with the colourisation sensors may therefore be used to determine the number of sweat glands at the sample areas. A colourisation sensor may be disposed in some or all of the cavities of the sample areas. The colourisation of the cavities may be monitored with respect to time, such that the colourisation of cavities is compared over the same time period in order to distinguish between colourisation of an adjacent cavity due to multiple sweat glands and colourisation of the adjacent cavity due to an extended period of sweat excretion. That is, the colourisation of the zero excretion rate (zero rate), base excretion rate (base rate) and multiples of the base excretion rate (multiple rate) are determined over the same time period.

Cavities in which no colourisation has occurred over the time period may be categorised as zero rate and it may be determined that no active glands are present at those sample areas. If colourisation occurs at a single cavity then the cavity may be categorised as base rate and it may be determined that a single active sweat gland is present at the sample area. Multiple adjacent colourised cavities may be categorised as multiple rate and it may be determined that there are multiple active sweat glands at the location, with the number of sweat glands equal to the number of adjacent colourised cavities. For example, if two adjacent cavities have colourised in the same time that a base rate cavity has colourised, then the adjacent cavities have filled twice as fast and it may be determined that there are two active sweat glands at the location of adjacent sample areas. Similarly, if there are three adjacent colourised cavities, then it may be determined that there are three active sweat glands at the location. Observation of the colourisation of cavities may therefore be used to determine the number of sweat glands at each sample area, and thus the total number of sweat glands covered by the fluidic structure.

The colourisation sensor may be disposed at the top of the cavity, so that the sweat interacts with the sensor and colourisation occurs when the cavity is full. The colourisation may be considered as an activation of a colour change caused by the dissolving of reagents and so it may be determined that a cavity is full when the colour of the cavity changes. The fluidic structure may be optically observed to identify the colourisation of cavities and so the sensor data may be optical data acquired from observation of the structure. The sensor may, for example, be a camera and the sensor data may, for example, be video data from the camera.

The processor may be further configured to: determine the sweat excretion rate at each sample area other than sample areas categorised as zero excretion rate by dividing the volume of the cavity of the sample area by the time taken for the colourisation to occur at the sample area; determine the total sweat excretion rate of the sample areas by summing the determined sweat excretion rate at all sample areas other than sample areas categorised as zero excretion rate; determine the total number of sweat glands by summing the determined number of glands at each sample area; and determine the sweat excretion rate per sweat gland by dividing the total sweat excretion rate by the total number of sweat glands.

The flow rate of the excreted sweat may therefore be determined by monitoring the time taken for the colourisation to occur. That is, if the colourisation sensor is disposed in the cavity such that it is activated when the cavity is full, then the time taken for the cavity to be filled may be observed. This may then be used in combination with the known volume of the cavity to determine the flow rate of the excreted sweat per sample area. The total excretion rate may be determined by adding the determined flow rates of all of the sample areas (that are not categorised as zero rate) and the average excretion rate per sweat gland may be determined using the total excretion rate and the determined total number of sweat glands.

The present invention extends to method aspects corresponding to the system aspects.

In particular, according to an embodiment of a second aspect, there is provided a method for determining a sweat excretion rate per sweat gland of a user, the method comprising: collecting, by a fluidic structure, sweat excreted at the surface of the skin of the user from sweat glands, by gathering excreted sweat at a plurality of sample areas of the fluidic structure in a cavity, each sample area comprising a cavity; acquiring sensor data from two or more sensors at one or more of the plurality of sample areas, the sensor data relating to an excretion rate of the excreted sweat at the sample area; receiving, by a processor, the sensor data from two or more of the sensors of the fluidic structure; determining from the received sensor data, the number of sweat glands at each sample area and a total sweat excretion rate of the sample areas; determining a total number of sweat glands from the number of sweat glands at each sample area; and determining a sweat excretion rate per sweat gland using the total sweat excretion rate and the total number of sweat glands.

According to an embodiment of third aspect, there is provided a fluidic structure for use in a system. The fluidic structure is configured to be in contact with the skin of the user to collect sweat excreted at the surface of the skin of the user from sweat glands, and the fluidic structure comprises: a plurality of sample areas, each sample area comprising a cavity configured to gather excreted sweat, and two or more of the plurality of sample areas comprising a sensor configured to acquire sensor data relating to an excretion rate of the excreted sweat at the sample area.

Embodiments of the present invention therefore extend to a fluidic structure for use in a system for determining a sweat excretion rate of a user and a system comprising the fluidic structure. Features of the first aspect apply to the third aspect mutatis mutandis, and vice versa.

Aspects of the invention may be implemented in digital electronic circuitry, or in computer hardware, firmware, software, or in combinations of them. Aspects of the invention may be implemented as a computer program or computer program product, i.e., a computer program tangibly embodied in an information carrier, e.g., in a machine-readable storage device or in a propagated signal, for execution by, or to control the operation of, one or more hardware modules. A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a communication system environment. A computer program may be deployed to be executed on one module or on multiple modules at one site or distributed across multiple sites and interconnected by a communication network.

Aspects of the method steps of the invention may be performed by one or more programmable processors executing a computer program to perform functions of the invention by operating on input data and generating output. Aspects of the apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

It may therefore be seen that embodiments of the present invention may provide means for determining the sweat excretion rate per sweat gland of a user. Determination of the average excretion rate per sweat gland allows for further analysis of the excreted sweat to be accurately performed and biomarkers in excreted sweat that vary as a function of the excretion rate per gland may be accurately assessed and analysed.

BRIEF DESCRIPTION OF THE DRAWINGS

Embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.

Figure 1 is a block diagram of a system according to a general embodiment of an aspect of the invention;
Figure 2 is a flow chart of a method according to a general embodiment of the invention;
Figure 3 is a diagram of a top view of a fluidic structure according to an embodiment of an aspect of the invention;
Figure 4 is a graph of the probability of a number of glands being present in a sample area;
Figure 5 is a diagram of a side view of a sample area according to an embodiment of an aspect of the invention;
Figure 6 is a diagram of a side view of a sample area according to an embodiment of an aspect of the invention;
Figure 7 is a diagram of a side view of a sample area according to an embodiment of an aspect of the invention;
Figure 8 is a diagram of a top view of a sample area according to an embodiment of an aspect of the invention;
Figure 9 is a graph of the determined velocity of sweat flow with respect to time according to an embodiment of an aspect of the invention;
Figure 10 is a diagram of a sample area according to an embodiment of an aspect of the invention;
Figure 11 is a diagram of a top view of a fluidic structure according to an embodiment of an aspect of the invention;
Figure 12A is a diagram of a cross-section view of a de-bubbler according to an embodiment of an aspect of the invention;
Figure 12B is a diagram of a side view of a de-bubbler according to an embodiment of an aspect of the invention;
Figure 13 is a diagram of a top view of a fluidic structure according to an embodiment of an aspect of the invention;
Figure 14A is a diagram of a fluidic structure according to an embodiment of an aspect of the invention;
Figure 14B is a diagram of a fluidic structure according to an embodiment of an aspect of the invention;
Figure 15A is a diagram of a fluidic structure according to an embodiment of an aspect of the invention;
Figure 15B is a diagram of a fluidic structure according to an embodiment of an aspect of the invention;
Figure 15C is a diagram of a fluidic structure according to an embodiment of an aspect of the invention; and
Figure 15D is a diagram of a fluidic structure according to an embodiment of an aspect of the invention.

DETAILED DESCRIPTION OF EMBODIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.
It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.
Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein may be used in the practice or testing of the embodiments.

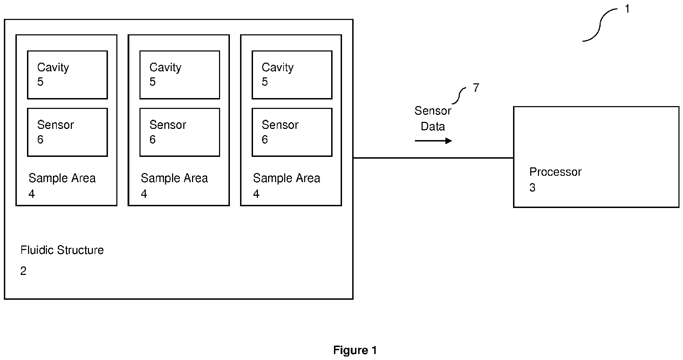

Figure 1 is a block diagram of a system for determining a sweat excretion rate per sweat gland of a user according to a general embodiment of an aspect of the invention. The system 1 comprises a fluidic structure 2 and a processor 3. The fluidic structure 2 comprises a plurality of sample areas 4, with each sample area 4 comprising a cavity 5 and at least two sample areas 4 comprising a sensor 6. That is, each sample area 4 has a cavity 5 and some or all of the sample areas 4 have a sensor 6. The arrangement of the fluidic structure 2 is shown in Figure 1 only as an exemplary block diagram to aid understanding, and the components of the fluidic structure may be arranged differently, in accordance with embodiments of the present invention. For example, a sensor 6 may be disposed in its respective cavity 5 or multiple sensors 6 may be present in one sample area 4. The fluidic structure 2 is connected to the processor 3. Sensor data 7 acquired by the sensors 6 and relating to an excretion rate of the excreted sweat at the respective sample area 4 is transferred between the fluidic structure 2 and the processor 3 for processing at the processor 3.

The specific type of sensor data may be determined by the type of sensor. For example, if the sensor is a flow rate sensor, then the sensor data may be a measured flow rate at each of the respective sample areas. The sensor data may include all of the sensor readings acquired from the sensors of the fluidic structure or it may include only some of the sensor readings. That is, it may only include sensor data for a subset of the sensors in the structure or may only include sensor data measured at a specific time point or during a specific period of time. The processor (data processing unit) receives the data from the plurality of flow rate sensors, and assesses the flow rate per gland, involving the assessment of the number of glands (excreting sweat) covered by each sample area based on a comparison and a discretization of the plurality of flow rates respectively measured at a time t at the plurality of sample collecting areas.

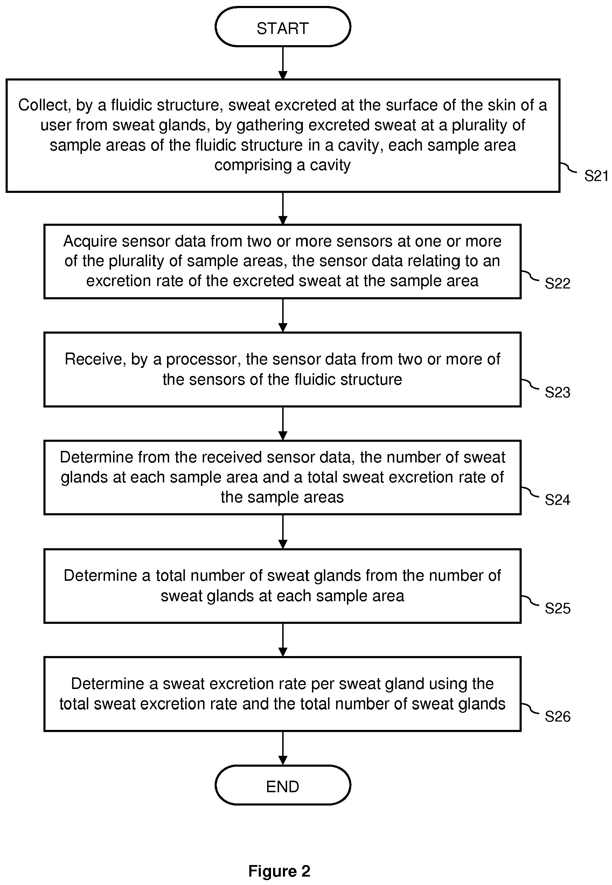

Figure 2 is a flow chart of a method for determining a sweat excretion rate per sweat gland of a user according to a general embodiment of the invention. At step S21, sweat excreted by sweat glands at the surface of the skin of the user is collected by the fluidic structure. More specifically, the excreted sweat is gathered in cavities of the fluidic structure at the plurality of sample areas. Sensor data is acquired from two or more sensors of the plurality of sample areas at step S22. The sensor data relates to an excretion rate of the excreted sweat at the sample area. The sensor data is then transferred to the processor, such that the processor receives the sensor data from two or more of the sensors of the fluidic structure at step S23. At S24, from the received sensor data, the number of sweat glands at each sample area and a total sweat excretion rate of the sample areas are determined. That is, the number of sweat glands at each sample area for which sensor data is received may be determined and the total sweat excretion rate may be the sum of the received sensor data indicating a sweat excretion rate at the sample areas. If no sensor data for a sample area is received then it may be considered that the sample area has no active sweat glands and no sweat excretion. A total number of sweat glands is then determined from the number of sweat glands at each sample area at step S25. This may be determined by summing the number of sweat glands at each sample area, as determined in the previous step. Finally, at step S26, a sweat excretion rate per sweat gland is determined using the determined total sweat excretion rate and the total number of sweat glands. This may, for example, be determined by dividing the total sweat excretion rate by the total number of sweat glands.

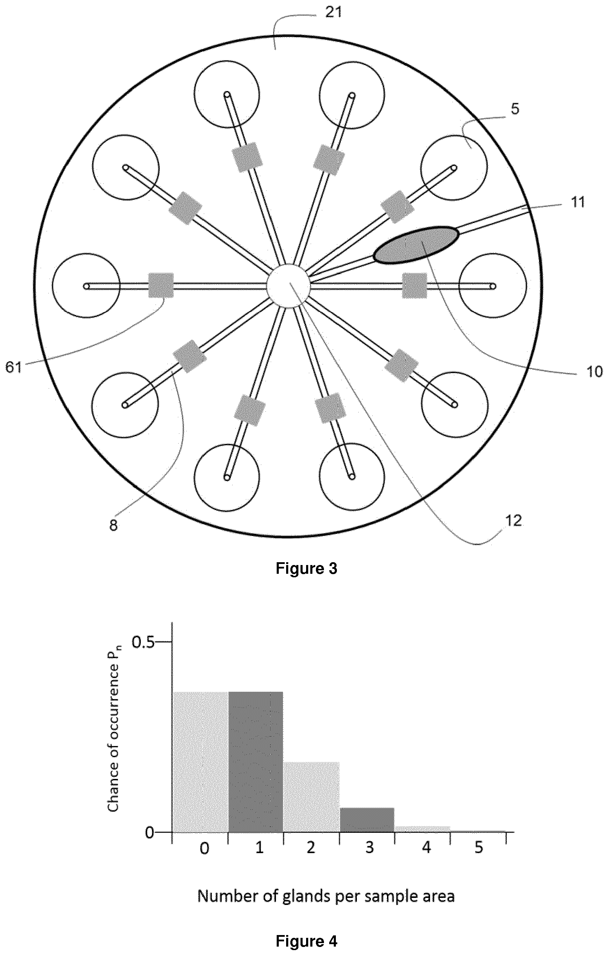

Figure 3 shows a diagram of a top view of a fluidic structure according to an embodiment of an aspect of the invention. The fluidic structure 21 is patch-like for attachment to the skin of the user and comprises multiple cavities 5, multiple sensors 61, multiple channels 8, a primary intersection 12, an exit channel 11 and a concentration sensor 10. The cavities 5 are each connected to the primary intersection 12 by the channels 8 and each channel 8 comprises a sensor 61. From the primary intersection 12, the exit channel 11 extends to the edge of the structure 21 to remove the sweat from the structure 21 and the concentration sensor 10 is disposed in the exit channel 11 to measure the concentration of a biomarker in the sweat before it exits the structure 21.

Ten sample areas are depicted in Figure 3 provided on one patch like fluidic structure. The sample areas may have a diameter of 1130 micron and an average height of 5 micron. All of the sample areas may have their own flow velocity sensor. The flow of all the sample areas is combined and as such will flow over a sensor determining the concentration of a particular compound in the sweat. The patch like fluidic structure may have a diameter of about 9 mm which is an acceptable size for a patch. A multitude of concentration sensors, each determining a different compound, may also be placed in the exit channel. After the concentration sensor the exit channel guides the sweat to the edge of the structure where a solution may be provided to remove the sweat; for instance by an absorber pad (not shown).

The number of sample areas in the fluidic structure may vary depending on the application of the invention, such as, for example, the location on the user at which the structure is positioned. In any case, the total sampling area of the device (i.e. the total surface area of the skin covered by the fluidic structure) is relatively small, whereby there may be considerable random variation in the number of sweat glands being sampled depending upon the exact position chosen to attach the device. In principle the parts of the fluidic structure may be the same for different arrangements, with the dimensions and the number of sample areas varying. It is evident that more sample areas/sample chambers may be constructed in one patch and that the sample areas may be increased by a factor of two in surface area.

First an example of the fluidic structure with many sample areas is discussed. The many sample areas still collectively, however, fall within the total sampling area of the device which remains relatively small. This example uses many sample areas each with a surface area that is sufficiently small to ensure that the probability that the number of glands per sample area is either zero or one is high. The average number of sweat glands per surface area does vary per body location but, in this example, a typical number of one active gland per square millimetre is used. Note that typically about 5-15% of the glands per surface area are active and are producing sweat. The other glands are dormant. Furthermore, it is reasonable to assume that the distribution of sweat glands at a particular body location is randomly distributed.

To calculate the required surface area per sample area, a Poisson distribution may be used, as follows:

$$P_n = \left[\frac{<n>^n}{n!}\right] \times exp^{(-<n>)}$$

Where $n$ is the number of glands, $P_n$ is the probability that $n$ glands are present in a particular sample area and $< n >$ is the average number of glands in the particular sample area.

So, if it is desired that the majority of sample areas only cover zero or one gland, $P_0 + P_1$ should be close to one. Theoretically seen this would mean that the sample area would be impractically small. Therefore, it is first considered that $P_0 + P_1$ should be 0.995. In this case, the chance that there would be two or more glands in a sample area would be 0.5%, which will have a minor effect on the accuracy of the excretion rate. The below example assumes that there is on average 0.1 active glands in a sample area ($< n > = 0.1$). This means that, with an average of 1 gland per square millimetre, the sample area should be 0.1 square millimetre. If a sample area is of circular shape, the diameter would be ≈360 micrometre resulting in a total area of ~0.1mm$^2$.

Note that the number of glands per surface area varies between body locations. For example, the number of glands per square millimetre at a finger is on average about 5.3, at the forehead is on average about 2.2 and is about 1 for the back (lumbar). Several other body locations are around 1 or lower. In the described examples, an average number of 1 gland/mm$^2$ is utilised. However, it may be seen that embodiments of the invention are applicable to other average glands numbers per surface area and it is only the size of the sample areas that may have to be adapted to optimise the structure for the average number of glands per surface area.

In this example where $< n > = 0.1$, the following may be calculated:

$$P_0 = \left[\frac{0.1^0}{0!}\right] \times exp^{(-0.1)} \approx 0.905$$

$$P_1 = \left[\frac{0.1^1}{1!}\right] \times exp^{(-0.1)} \approx 0.090$$

$$P_0 + P_1 \approx 0.995$$

If only one such sample area is provided, then the chance that no excretion is measured is 90.5% ($P_0$). This constitutes an enormous, unacceptable false negative number. If the threshold for false negatives is set to 0.01%, at least 93 of these sample areas would be required in a skin patch. In this case the chance of having only sample areas with zero glands is 0.905$^{93}$≈ 0.0001 which is 0.01%. For a false negative threshold of 1%, at least 46 of sample areas of 0.1mm$^2$ would be required (0.905$^{46}$≈0.01).

93 sample areas each with a surface area of 0.1mm$^2$ would result in a total surface area of the structure of ≈28mm$^2$. This is because it is assumed that only a third of the surface area of the structure is actually formed from the sample areas and the remaining surface area is formed from borders and channels around and between the sample areas. When the fluidic structure is provided as a patch, 28mm$^2$ constitutes a reasonable size of the sample collection area, i.e. a reasonable size of the patch. However, such an arrangement would also require 93 sensors monitoring the excretion rates of the individual sample areas if a sensor is disposed in each sample area. When using, for example, an optical sensor array, this is acceptable (it is known for CCD cameras to have sensors with more than a million pixels). Alternatively, with MEMS technology, very small flow rate sensors are able to be made based on thermal distribution. The determination of the excretion rate per gland is further illustrated in the detailed discussion below.

So the above mentioned example is within the range of embodiments of the present invention yet may be technically challenging, for example, care has to be taken that no leakage will occur between the sample areas. However, by using a statistical algorithm based on the Poisson distribution, the number of required sample areas may be reduced and the manufacture of the fluidic structure may be simplified with increased reliability.

A second example of the fluidic structure with fewer sample areas will now be discussed. In this example, there is an average of 1 active gland in a sample area (<n>=1), which is ten times greater than in the previous example. This means that, with an average of 1 gland per square millimetre, the sample area should be 1 square millimetre, i.e. ten times larger than the sample area of the previous example. If the sample area has a circular shape, then the required diameter of one sample area would be ≈1130 micrometre.

In this case, < n > = 1 and so the following may be calculated:

$$P_0 = \left[\frac{1^0}{0!}\right] \times exp^{(-1)} \approx 0.368$$

$$P_1 = \left[\frac{1^1}{1!}\right] \times exp^{(-1)} \approx 0.368$$

$$P_2 = \left[\frac{1^2}{2!}\right] \times exp^{(-1)} \approx 0.184$$

$$P_3 = \left[\frac{1^3}{3!}\right] \times exp^{(-1)} \approx 0.061$$

$$P_4 = \left[\frac{1^4}{4!}\right] \times exp^{(-1)} \approx 0.015$$

$$P_5 = \left[\frac{1^5}{5!}\right] \times exp^{(-1)} \approx 0.003$$

$$P_0 + P_1 \approx 0.736$$

The calculated probabilities may be represented graphically, as shown in Figure 4. If only one such sample area is used, then the chance that no excretion is measured is 36.8%. This would still constitute an enormous, unacceptable false negative number. If the threshold for these false negatives is again set to 0.01%, only 10 of these sample areas would be required in the structure (the chance that all sample areas have zero glands is $0.368^{10} \approx 0.0001$, which is 0.01%).

Assuming again that only a third of the area of the structure is made up of the sample areas, the total surface area of the fluidic structure would be ≈30 square millimetres. That is, 10 sample areas each with a surface area of $1mm^2$ would result in a total surface area of the structure of ≈$30mm^2$. For a structure provided as a patch this constitutes a reasonable size of the sample collection area. It would also mean that only 10 sensors monitoring the excretion rates of the individual sample areas are required if each sample area comprises a sensor. When using 10 simple optical sensors, an optical sensor array may not be required, saving cost and space. Other sensors may also be considered, such as electrodes measuring impedance, or sensors based on thermal distribution measuring flow velocity. If the acceptable probability of measuring no excreted sweat is reduced to 1%, then only 5 sample areas will be required.

The following example for demonstrating the measurement of the excretion rate per gland utilises 10 sample areas. If it is first assumed that all glands excrete the same amount of sweat, which will be called x. From the calculated probabilities ($P_n$) above, it may be derived that about 3-4 sample areas will excrete zero, about 3-4 sample areas will excrete x and about 1-2 sample areas will excrete 2x and about 0-1 sample areas will excrete 3x and, in rare cases, it may be found that a sample area excretes 4x. The chance that an excretion of 5x or even more would be found is very rare.

Since the lowest value x is known to be caused by one gland, x may be determined. All channels with glands may be used to determine the average excretion per gland, since it is known that each sample area contains one or a multitude of glands. In this sense, the method could be typified as a discretization method because is relies on differen-

tiation between *x, 2x, 3x,* etc. This process is illustrated by the example shown below, in which it is seen that there is a typical variation of about 10% on the excreted amounts per individual gland. Table 2 below shows example detected excretion rates for the ten sample areas.

*Table 2: Excretion rates for the sample areas of the example*

| Sample Area Indicated by Number | Measured Excretion Rate in nl/min |
|---|---|
| 1 | 2.2 |
| 2 | 2.0 |
| 3 | 6.6 |
| 4 | 0.01 |
| 5 | 4.0 |
| 6 | 4.4 |
| 7 | 0.005 |
| 8 | 3.8 |
| 9 | 0.01 |
| 10 | 2.3 |

In this example, it is assumed that the person carries out modest exercise and that the excretion rate of sweat glands is in the order of nanolitres/min per gland. Values much smaller than 1 nl/min may be attributed to a sample area with no glands and the measured negligible value may be regarded as noise. Therefore, in this example, it may be concluded that sample areas 4, 7 and 9 contain no active glands. These sample areas are omitted from further calculations.
The sample areas with the lowest excretion values are next looked at, and these excretion rates are attributed to one gland. In this case, it is concluded that sample areas 1, 2 and 10 each contain one gland. The excretion rate for one gland is therefore about (2.2 + 2.0 + 2.3)/3 ≈ 2.167 nl/min.
Subsequently, sample areas with excretion rates that are approximately double that of the excretion rate of one sweat gland are identified as sample areas with two glands. In this example, the excretion rate is approximately 4.4 nl/min and the excretion rates of sample areas 5, 6 and 8 are each attributed to two glands. Next sample areas with excretion rates that are approximately three times larger than that of the excretion rate of one sweat gland are identified as sample areas with three glands. The excretion rate in this example is about 6.6 nl/min and sample area 3 is attributed to three 3 glands. It may therefore be considered that the excretion rate of one gland is the largest common divisor of the other measured excretion rates (other than those at or close to zero).
From this analysis, it may be calculated that there are twelve active glands in total covered by the ten sample areas. That is, there are three sample areas with no active sweat glands, three sample areas with one active sweat gland each, three sample areas with two active sweat glands each and one sample area with three active sweat glands, providing a total of twelve sweat glands. The average excretion rate may then be calculated using the determined total number of glands and the measured excretion rates at each sample area that is determined to contain at least one gland. The calculation of the average excretion rate per gland for the above example is as follows:

$$\frac{(2.2 + 2.0 + 2.3 + 4.0 + 4.4 + 3.8 + 6.6)}{12} \approx 2.108 \, nl/min$$

The allowed variation in the measured excretion rate per sample area should preferably be such that overlap between the excretion rates of one gland, two glands and three glands does not occur. Considering that the surface area of a patch is ~1 cm$^2$ or smaller and that sweat glands are close to each other, it is reasonable to assume that the eccrine glands (the coiled portion of the gland below the surface of the skin) are of similar size and that the sweat rate is similar. Even with a random variation on the order of 50-100% with respect to the measured excretion rate, the average sweat rate per gland may still be accurately determined. It is known that sweat glands may vary in size between different areas of the body, yet embodiments of the present invention employ local sweat glands. This variation has two sources: measurement error when measuring the excreted sweat rate at the sample areas; and the variation in excretion rate per gland.
Of course, due to randomness in the positioning of active glands, the number of samples areas with one gland, with two glands etc. will vary. However, the chance that none of the sample areas cover one gland is low: $(1-0.368)^{10} \approx 0.01$, which is about 1%. So in 99% of the cases, the excretion rate of one gland may be identified. However, for the sake of completeness, if there are no sample areas that cover one gland, then the lowest excretion rate that would be found would be *2x*. This could be falsely interpreted as *x,* and so the sample areas with two glands would be falsely identified as sample areas with one gland. However, in that case, the sample areas containing 3x would be identified as having 1.5 glands, i.e. the measured excretion rate would be approximately 1.5 times the lowest excretion rate. There may be no partial or fractional sweat glands and so the determination of 1.5 glands would be known to be an error and so it may be deduced that the excretion rate identified as x is in fact 2x. The average excretion rate per gland may then be accordingly determined.

It is noted that glands half covered by a rim (the edge) of a sample area will still excrete almost the same and the hydrodynamic resistance is only influenced in a minor fashion by partially blocking the duct (a small part of the total hydrodynamic resistance of the duct). Furthermore, the occurrence of glands on the rim is rather low. It is preferable that the spacing between sampling areas is at least as wide as a typical gland in order to minimise the chance of a sweat gland being positioned at the edge of a cavity and the sweat being spread over two sampling areas. It may also be advantageous to have hydrophobic material between the sample areas.

In rare situations, it may not be possible to definitively determine the number of active sweat glands. For example, a scenario in which zero sweat flow rate is measured in some sample chambers, A nl/min sweat flow rate is measured in other sample chambers and 2A nl/min is measured in other sample chambers shall be considered. In this example, a first configuration could be interpreted, in which the excretion A nl/min is the excretion of one sweat gland and the excretion 2A nl/min is the excretion of two sweat glands. However, a second configuration could also be interpreted, in which A nl/min is the excretion of two glands and 2A nl/min is the excretion of four glands. It may not be possible to distinguish between these configurations but the chance that the selected configuration is incorrect may be determined. For example, if the first configuration is selected, then the chance that this is incorrect is equal to the chance that the second configuration is true. For this example, where the average sweat gland number per sample area is 1, the chance of occurrence of the second configuration may be statistically calculated and is -0.0008. There may be other configurations which may not be distinguished and all these chances have to be added. Again with statistical calculation, the chance of a wrong interpretation may be approximated, which is -0.0036 (always assuming that the lowest non-zero value is excreted by one gland). In other words, there is a 0.36% chance that the average sweat flow rate per gland is over estimated, which may constitute a false negative. The expected flow rate at the location at which the structure is positioned may be used to increase the possibility of selecting the correct configuration.

The false negative number of the structure may be decreased, as discussed above, by increasing the number of sample areas. Alternatively, by increasing the sample area size, the number of sample chambers may be decreased. When the sample area is two times larger in surface area, the required number of sample chambers is approximately half. The flow rate per sweat gland may still be derived. However, as the size of the sample areas increases, the differentiation between the numbers of glands per sample area will get increasingly more difficult, especially considering noise in the measurement. An effective structure will therefore provide a suitable balance between the number of sample areas and the size of the sample areas. The inventors found that approximately 13 sample areas (with discretized sensor elements) per patch are sufficient to determine the number of glands per surface area in an efficient manner and to determine the excretion rate per gland. Moreover, they found that the optimal number of glands per sample area should be 1 to 2 glands on average. The number of sample areas may vary from 5 to 100. The average number of glands per sample area may vary between 0.1 and 5. The difficulty of the discretization of the method increases as the number of glands per sample area increases, especially considering noise in the measurements.

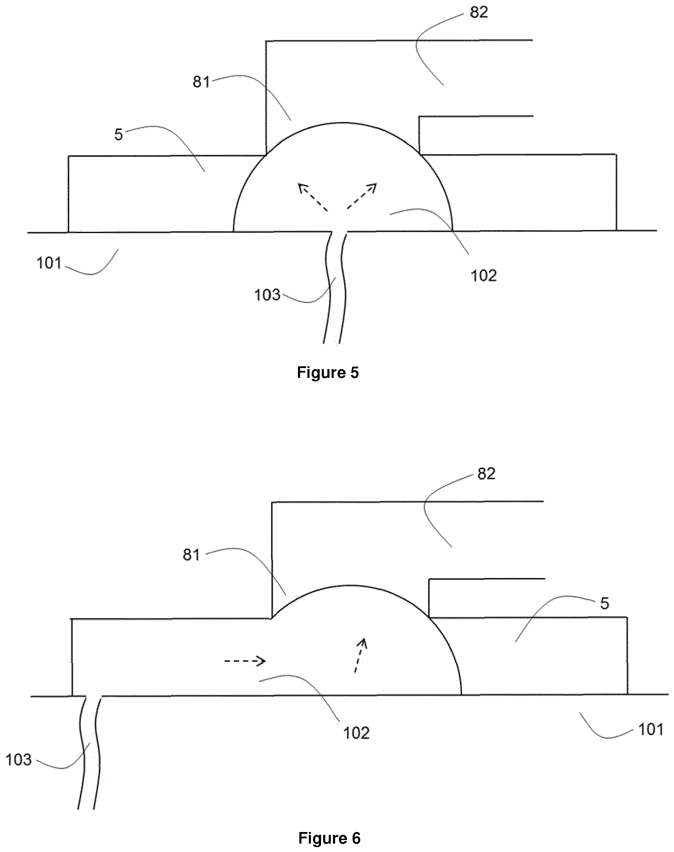

Figure 5 shows a diagram of a side view of a sample area according to an embodiment of an aspect of the invention. The sample area comprises a cavity 5, a cylindrical channel 81 and a rectangle channel 82. The cavity is positioned to cover a portion of the skin 101 and sweat 102 is excreted from a sweat duct 103 of a sweat gland. In Figure 5, the material of the microfluidic system is hydrophobic, for example PDMS. Filling of the cavity in Figure 5 occurs from the sweat duct centred in the cavity until the sweat hits the cylindrical channel.

As discussed in detail above, the design of the sample area may be altered based on the prima facie probability of finding more or less glands per sample collecting area. The cavity of Figure 5 may be considered as a sample collecting area construct of small surface area for measuring flow rate. In this case the cavity may be used with a small sample collecting area with a cavity of 360 $\mu$m diameter and 50 $\mu$m height. The sample collecting area is relatively small to provide the fluidic device with a large number of sample collecting areas, in case the chance of the presence of zero glands per sample area is high (e.g. 90%), the chance of the presence of one gland per sample area is much lower (e.g. 9%) and the chance of two or more glands is even lower (e.g. 1%). When using about, for example, 100 sample areas in the fluidic device, the data processing unit employing the sweat rate per cavity may derive the sweat rate per gland according to embodiments of the invention.

Figure 6 shows a diagram of a side view of a sample area according to an embodiment of an aspect of the invention. The sample area is the same as that shown in Figure 5 yet the position on the skin, with respect to a sweat gland, differs. Specifically, the sweat duct 103 in Figure 6 is located at the edge of the cavity 5 and is not positioned directly below the cylindrical channel 81. The cavity 5 is therefore filled by the sweat duct 103 close to the rim of the cavity 5 until the sweat 102 hits the cylindrical channel 81. The material of the sample in Figure 6 is a hydrophobic material.

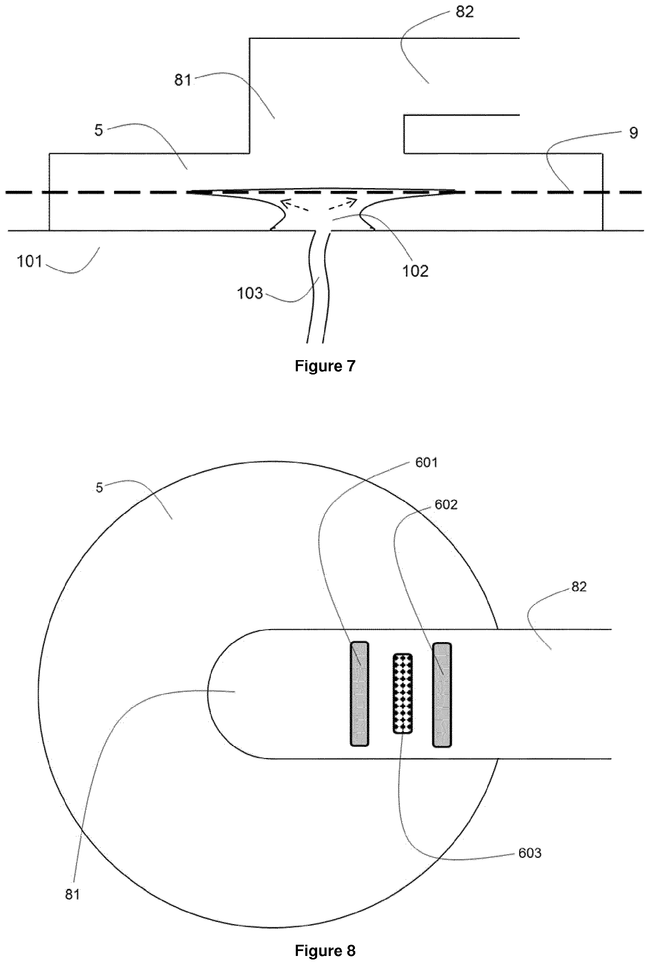

Figure 7 is also a diagram of a side view of a sample area according to an embodiment of an aspect of the invention with similar components. However, the sample area of Figure 7 further comprises a fluid leveller 9, which is a hydrophilic membrane. Furthermore, the material of the structure is hydrophilic, for instance coated PDMS, such that all surfaces except for the skin are hydrophilic. Sensor elements are not shown in any of Figures 5, 6 or 7.

The sample area of Figure 7 may be a sample collecting area construct with a large surface area for measuring the flow rate. In this case, the structure may have a larger sample collecting area with a cavity of 1130 $\mu$m diameter. The sample collecting area is relatively large with respect to the cavity of Figure 5 discussed above. The larger sample area is applicable when the prima facie probability of the presence of one (and/or two) glands per sample collecting area is substantially higher than for Figure 5 above. Nevertheless, due to the discretization (0, 1, 2, 3 glands per sample collecting area), the data processing unit only has to employ the sweat rate of a limited number of sample collecting areas (~10) to derive the average sweat rate per gland.

Table 3 below lists a number of parameters, which aid in the description of the structures. The capillary pressure of a circular channel is:

$$P_C = \Upsilon \times 2 \times \cos(\theta) / r$$

where $P_C$ is the capillary pressure in a channel with circular cross section with radius $r$ and a contact angle of $\theta$ and $\Upsilon$ is the surface tension. The capillary pressure of a rectangle channel is:

$$P_{Rec} = \Upsilon \times 3 \times \cos(\theta) / L_1$$

where $P_{Rec}$ is the capillary pressure in a channel with rectangle cross section with height $L_1$, a width of $2 \times L_1$ and a contact angle of $\theta$.

*Table 3: Parameters of PDMS, sweat and eccrine sweat glands.*

| **Input Parameters** | | |
|---|---|---|
| PDMS | Contact angle with water | 115 degrees |
| PDMS coated with 0.01% Dopamine | Contact angle with water | 60 degrees |
| Sweat | Surface tension | 70 * 10$^{-3}$ N/m |
| Circular channel | Diameter | 100 * 10$^{-6}$ m |
| Rectangle channel | Height | 50 * 10$^{-6}$ m |
| Rectangle channel | Width/height ratio | 2 |
| Sweat gland | Max pressure at 20 nl/min | 70 * 10$^3$ Pascal |
| Sweat gland | Pump | Pulsating |
| Sweat gland | Pulsation | Sweat event on average 30 seconds every 3 minutes |
| Cavity | Sample chamber | Diameter 360 micrometer Height 50 micrometer |
| | | |
| **Derived Values** | | |
| Sweat gland | Average excretion rate during the active period of the pulse cycle (30 seconds of 3 minutes) | 6 nl/min |
| Sweat gland | Assumed pressure at average low excretion rate per pulse cycle of 1 nl/min | 3.5 * 10$^3$ Pascal |

(continued)

| Derived Values | | |
|---|---|---|
| Pressure at hydrophobic contact angle of 115 degrees (PDMS, see above) | Circular channel | -1.18 * $10^3$ Pascal |
| | Rectangular channel | -1.77 * $10^3$ Pascal |
| Pressure at hydrophilic contact angle of 60 degrees | Circular channel | 1.4 * $10^3$ Pascal |
| | Rectangular channel | 2.1 * $10^3$ Pascal |

Note that a positive pressure stimulates flow into the microfluidics structure and negative pressure resists flow into the microfluidic structure. Note that the excretion rate is an average value over the pulsation cycle, so if the active time is 30 seconds and the total pulsation cycle is 3 minutes, the average excretion rate in the active 30 seconds period is six times more. Note that in Table 3 the hydrophobic channels have a somewhat negative capillary pressure but the pressure as generated by the sweat gland is larger, so sweat flow will occur.

As shown in Figure 5, the filling of the cylindrical channel commences in the fastest scenario after about 7.5 seconds, at the moment when the sweat volume in the cavity is about a half sphere with radius 72 micrometer, constituting a volume of 7.5 * $10^5$ cubic micrometer.

However due to the pulsating nature of the sweat gland (in estimate on average 30 seconds actively excreting sweat within a period of 3 minutes) there may be a delay of about 2.5 minutes for instance if the patch is attached just as the active event stops. In a worst case scenario, the flow velocity sensor may not be able to record a first measurement until after about 3 minutes have passed (about 2.5 minutes for the gland starting to eject again, 7.5 seconds for reaching the circular channel and 11 seconds to just pass the downstream sensor). As shown in Figure 6, the filling of the cylindrical channel may commence roughly after 3 * $10^6$ cubic micrometre which is 30 seconds after applying the cavity to the skin, due to the position of the channel with respect to the sweat duct. In the worst case, the flow velocity sensor will record a liquid flow velocity after about 6 minutes (about 2.5 minutes when the gland starting to eject again, 30 seconds for commencing filling of the cylindrical channel, again 2.5 minutes for the gland being in dormant state and 11 seconds for reaching the sensor). Moreover, it is handy to wipe the skin dry before attaching the patch (to ensure good adhesion and eliminate unknown excess sweat on the skin).

Figure 8 is a diagram of a top view of a sample area according to an embodiment of an aspect of the invention. The sample area comprises a cavity 5, a cylindrical channel 81, a rectangle channel 82 and a sensor 62. The sensor 62 is a flow velocity sensor that is disposed in the rectangle channel 82. The sensor 62 comprises an upstream temperature sensor 601, a downstream temperature sensor 602 and a pulsed heating element 603 (i.e., a thermal mass flow meter). The pulsed heating element (pulsed heater) 603 is disposed between the upstream temperature sensor 601 and the downstream temperature sensor 602.

The flow velocity is determined by calculating the difference between a temperature measured by the upstream temperature sensor 601 and a temperature measured by the downstream temperature sensor 602 with respect to the pulsed heating element 603. When sweat flows over the flow sensor and a pulse of heat is given, the downstream temperature sensor 602 will record a higher temperature with respect to the upstream temperature sensor 601. So the heated part of the sweat will flow over the downstream temperature sensor 602. However, due to some conduction, the upstream temperature sensor 601 will also record a slight temperature increase. The sensor 62 may be pre-calibrated in laboratory testing using different fluids.

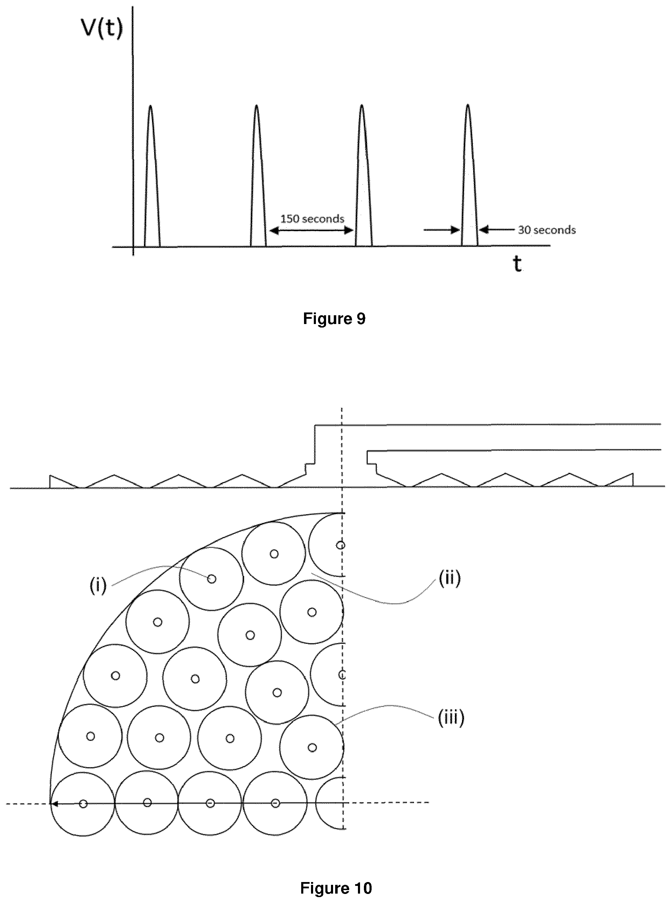

The difference in temperature recorded by the sensors is a measure for the flow velocity. So in the case of one active gland for 30 seconds and subsequently 2.5 min dormant state, a velocity profile will be measured as schematically shown in Figure 9. The peaks in the graph represent the periods when the gland is active and the spaces between the peaks represent the periods when the gland is dormant. The flow rate may be calculated from the recorded velocity as a function of time by integration. The integration time should preferably be a multitude of cycles, since the cycles may be easily observed and included in an algorithm.

The formula to calculate the flow rate is as follows:

$$Sweat\ excretion\ rate = \int V(t) \times \frac{CS}{nT}.dt$$

where V(t) is the recorded flow velocity as a function of time, CS is the cross-sectional area of the rectangle channel and nT a multitude of cycle times T. The integration is carried out over a time that equals nT.

It should be noted that, as more than one gland is present, the velocity profile may differ (for example, more peaks and/or overlapping peaks, glands having slightly different cycle times, etc). However, if nT is sufficiently large, an accurate sweat excretion rate may still be determined. For instance, assuming that one gland has a cycle time twice as long, then with five times this cycle time a reliable sweat secretion rate may be determined. The velocity profiles of two or even three glands may even be distinguished between and the individual sweat excretion rate could be determined from each gland velocity profile.

With reference to Figure 7 in which the material has a hydrophilic surface and hence the capillary pressure will support the flow through the channel. However, when a sweat droplet as created by the sweat gland hits the cylindrical channel, it may suddenly be aspirated from the hydrophobic skin surface and along the flow sensor. This may introduce a pulse in the flow sensor that is not related to the pulsing behaviour of the sweat gland. Although the device will remain functional in determining the average sweat flow rate, the additional information of the pulsating behaviour of the sweat gland may not be determined anymore. Consequently, to enable measuring the pulsating behaviour of the sweat gland, a fluid leveller in the shape of a hydrophilic flat-shaped membrane is placed in the middle of the height of the cavity. When the sweat hits this membrane the fluid will flow over the membrane and subsequently start to fill the upper part of the cavity with a rather flat meniscus, independently of the position of the gland. Such a fluid leveller could also be used in a hydrophobic cavity, such as that of Figure 5.

If the diameter of a cavity is 1130 micrometre and the height is 50 micrometres, then a complete filling of the cavity would take approximately 50 minutes with a sweat excretion rate of 1 nl/min for one gland. This would result in a serious delay in measuring the first sweat excretion rate. This problem may be resolved by simply decreasing the height of the cavity to 5 micrometres. However, the elasticity of the skin may close the cylindrical channel and thus close the cavity outlet. Consequently, conical pillars may be provided to prevent collapsing of the cavity by skin elasticity, whilst decreasing the volume of the cavity, for example, by a factor of ten to reduce the filling time to 5 minutes.

Figure 10 shows a diagram of a sample area according to an embodiment of an aspect of the invention in which the cavity is segregated into a plurality of conical pillars. The upper part of Figure 10 shows a side view of the sample area and the lower part of Figure 10 shows a bottom view. The upper part shows the cross-section of the centre of the cones in contact with the skin (indicated as at point (i) in the lower part of Figure 10). Between the cones the sweat may freely flow into the construct (indicated as points (ii) and (iii)) and, as may be seen from the lower part of the Figure, may flow freely towards the centre of the construct where the sweat may exit via the cylindrical channel, as shown in the upper part of the Figure.

As may be seen from Figure 10, the cavity is segregated into a plurality of conical pillars which provide, for example, an average cavity height of 5 micrometer resulting in a filling time of approximately 5 minutes. The material is a hydrophobic material but a similar structure may be used for a fluidic structure made of hydrophilic material. The diameter of the sample area in Figure 10 may be 1130 micrometer. The channels may be hydrophilic having a somewhat positive capillary pressure and would support the sweat flow.

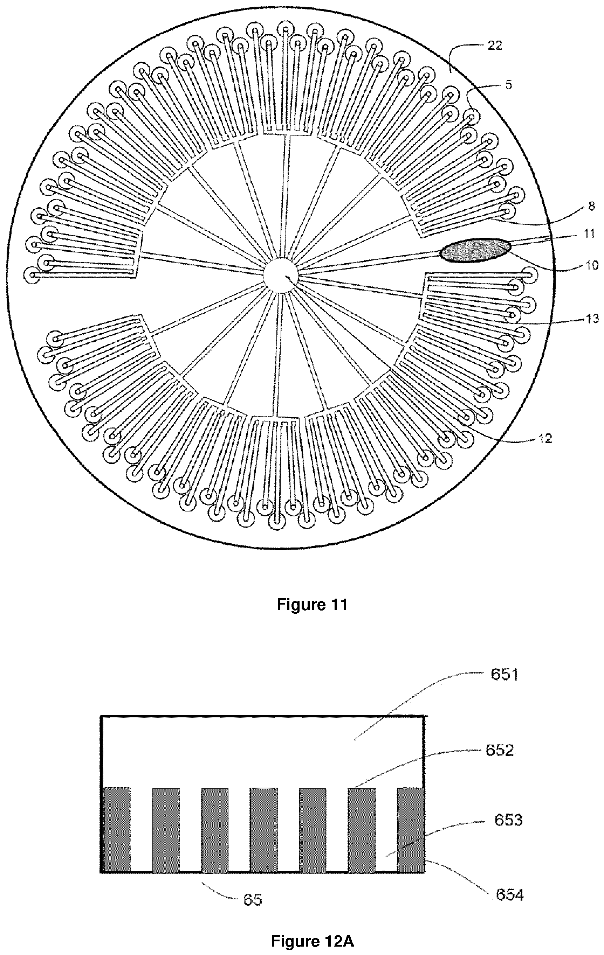

Figure 11 shows a diagram of a top view of a fluidic structure according to an embodiment of an aspect of the invention. The fluidic structure 22 is patch like for attachment to the skin of the user and comprises multiple cavities 5, multiple channels 8, a primary intersection 12, multiple secondary intersections 13, an exit channel 11 and a concentration sensor 10. Subsets of the cavities 5 are each connected to a secondary intersection 13, and each of the secondary intersections 13 are connected to the primary intersection 12. From the primary intersection 12, the exit channel 11 extends to the edge of the structure 22 to remove the sweat from the structure and the concentration sensor 10 is disposed in the exit channel 11 to measure the concentration of a biomarker in the sweat before it exits the structure 22.

The structure of Figure 11 has 96 sample areas provided on one patch like fluidic structure, with all of the sample areas having their own flow velocity sensor (not shown). The sample areas may each have a diameter of 360 micron. The flow of all the sample areas is combined and as such will flow over a sensor determining the concentration of a particular compound in the sweat. In this embodiment the patch-like fluidic structure has a diameter of about 12 mm which is an acceptable size for a patch. A multitude of concentration sensors, each determining a different compound, may also be placed in the exit channel. After the concentration sensor, the exit channel guides the sweat to the edge of the structure where a solution may be provided to remove the sweat; for instance, by an absorber pad (not shown).

At the points where flows of a number of sample areas are combined (the intersections), the sweat flow of one sample area may arrive earlier than that of another sample area (for example, there may be a different number of glands present in the sample area associated with one of the channels leading to a higher flow rate). This may temporarily induce some flow in the direction of the other sample area, i.e. flow away from the primary intersection. However, this would compress the air in this channel and the flow in the wrong direction would be stopped very quickly. Furthermore, the sweat flow as generated in the other sample area would quickly push the sweat again in the correct direction. The latter may, however, invoke an air bubble in the stream of the sweat flow towards the sensor determining the concentration of a particular compound to be determined (concentration sensor). Some sensors will be able to cope with such an air bubble, yet if the sensor cannot handle an air bubble, a de-bubbler may be provided. The de-bubbler may be positioned in any of the channels but preferably in the exit channel, towards the concentration sensor.

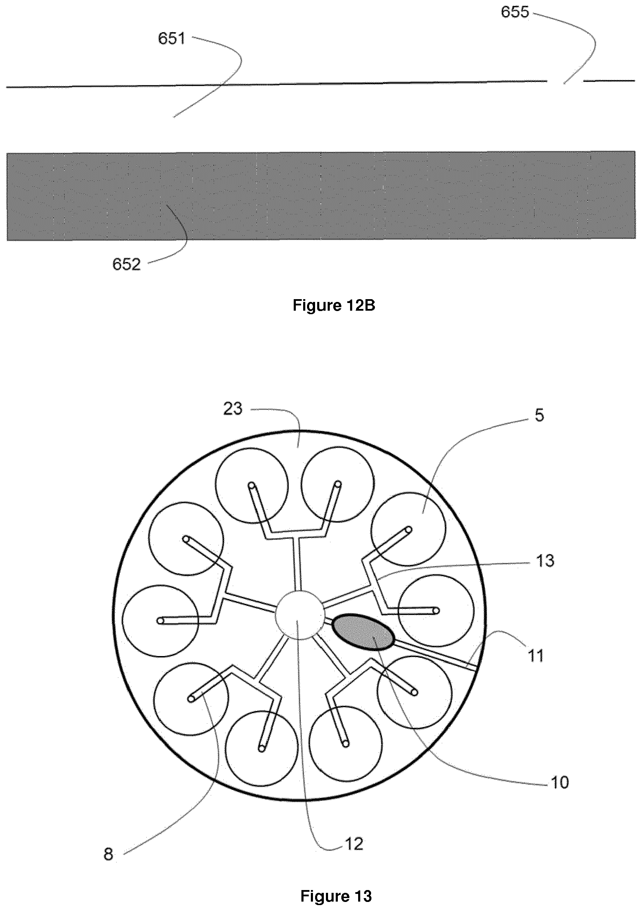

Figures 12A and 12B show diagrams of a de-bubbler according to an embodiment of an aspect of the invention.

Figure 12A shows a cross-section view of the de-bubbler and Figure 12B shows a side view. The de-bubbler 65 comprises a hydrophobic upper part 651, a hydrophilic lower part 652 and a vent 655, which may contain a hydrophobic membrane. The lower part 652 comprises a plurality of hydrophilic projections 654 arranged sequentially to define a plurality of channels (grooves) 653 between the hydrophilic projections 654. The air de-bubbler may be placed in the final channel towards the concentration sensor. The surfaces of the projections are made of hydrophilic material and the channel is vented by a small opening on the top of the channel 655.

When a concentration sensor is provided as part of the system, both the average sweat excretion rate per gland and a concentration of a particular compound in sweat, of which the concentration depends on the sweat rate, may be determined. Subsequently from a graph displaying the concentration of a particular biomarker as function of the sweat rate per gland (which may be determined using healthy volunteer subjects), it is possible to read the expected concentration of the biomarker at a measured sweat rate per gland for a healthy person. If the measured concentration at the measured sweat rate per gland deviates from this normal value, the presence of a (clinical) disorder related to the particular biomarker may be determined.

Depending on the position of the concentration sensor, there may be a delay between measuring the flow velocity of sweat per sample area and the measuring of the concentration (by the concentration sensor). However, since the flow is known, it is possible to correlate the correct sweat flow rate to the moment of measuring the concentration. Alternatively, a 'cauliflower' arrangement of the structure may be provided which shortens the length between the sample areas and the concentration sensor. Moreover, due to the early combination of a number of sample areas, the flow rate is increased after the combination of these flows, shortening the delay between velocity measurement and concentration measurement. The diameter of the sample area may be 1130 micron.

The 'cauliflower' arrangement of the fluidic structure is shown in Figure 13. The fluidic structure 23 comprises multiple cavities 5, multiple channels 8, a primary intersection 12, multiple secondary intersections 13, an exit channel 11 and a concentration sensor 10. Pairs of the cavities 5 are each connected to a secondary intersection 13, and each of the secondary intersections 13 are connected to the primary intersection 12. Alternatively or additionally to the arrangement of Figure 13, the fluidic structure may comprise multiple concentration sensors, each located in the vicinity of a flow rate sensor, so as to minimise the delay between flow rate sensing and concentration sensing.

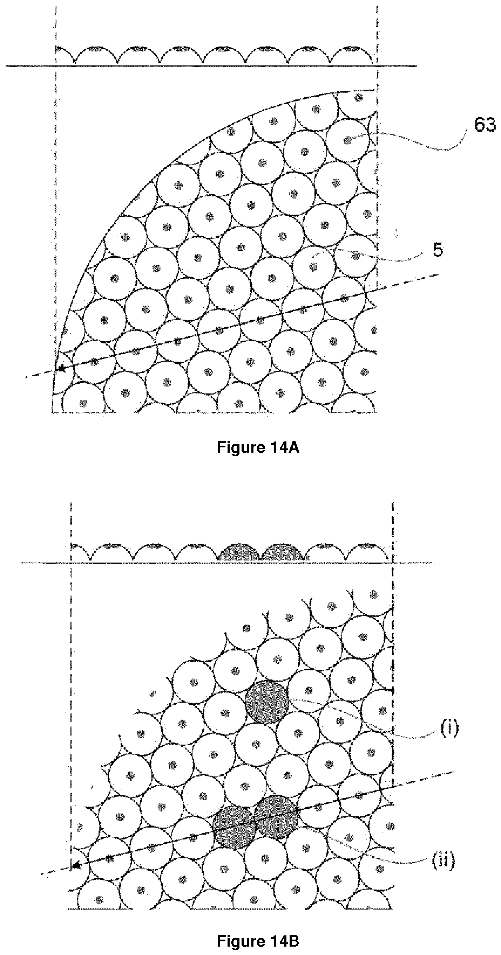

Figures 14A and 14B are diagrams of a fluidic structure according to an embodiment of an aspect of the invention. The fluidic structure uses sensors that record the presence of sweat. In the cavity of each sample area, a sensor is placed at the top of the sample area cavity (based on e.g., a colorimetric or an electrochemical measurement principle). As with the other embodiments, each sampling area is such that the either 0, 1 or a multitude of glands are underneath the area. The general design of such a device is depicted in Figure 14A, which shows a bottom view of the structure, with a side view. A plurality of cavities 5 are arranged directly adjacent to each other, with a colorimetric sensor 63 disposed in each cavity. The cavities 5 are connected in such a manner that when a sample cavity 5 is filled, additional fluid may migrate to an adjacent cavity.

When a gland underneath a sampling area is active, it will fill the cavity until the sensor is activated. In case of a colorimetric design, the reagents are dissolved and a colour change of the cavity may be optically observed. By linking the sampling areas around the first sampling area the amount of sweat glands underneath of the sampling area may be derived. In the case that, for example, two glands are present in a sample cavity, this cavity will fill faster than cavities containing one or zero glands and will overflow into an adjacent cavity. Figure 14B shows the colouration of cavities of the structure. Figure 14B shows the filling of cavities with sweat over the same time period. At point (i), one cavity has changed colour and so one gland has filled the sample cavity. At point (ii), two adjacent cavities have changed colour in the same time period and so the sample cavity was filled by two glands and the sweat overflowed to an adjacent sample cavity. It is therefore possible to determine that there is one sweat gland at position (i) and two sweat glands at position (ii).

In other words, in the case that, for example, two glands are present in a sample cavity, this cavity will fill faster and will overflow faster to an adjacent cavity, as schematically indicated in Figure 14B. By recording the sensor responses (for instance by a camera monitoring a colour change) and using a dynamic analysis of these responses, the average flow rate per gland can be determined, using the same algorithms as discussed in the in the above described embodiments.

By recording the sensor responses (for instance by a camera monitoring a colour change) and using a dynamic analysis of these responses, the average flow rate per gland may be determined using the same algorithms discussed in detail above. The snapshot as represented in Figure 14B (depending on the dimensions and the pulsed sweat production) reveals the areas where only one sweat gland is contributing to the sensor signal or where multiple sweat glands are contributing to the signal. In addition, the ratio of active sweat glands may be determined by counting the amount of 'active sensors' and 'inactive sensors'. The size of the cavities and the time taken for the cavities to fill may be used to determine the flow rate of the sample areas.

The structure of Figures 14A and 14B is simplified from a manufacturing point of view since no microfluidic channels are needed and no electrical wiring in the device is required when a colouration sensor is used. The sensor component may be added by inkjet print (or similar) techniques or silk-screening (for example printable electronics).

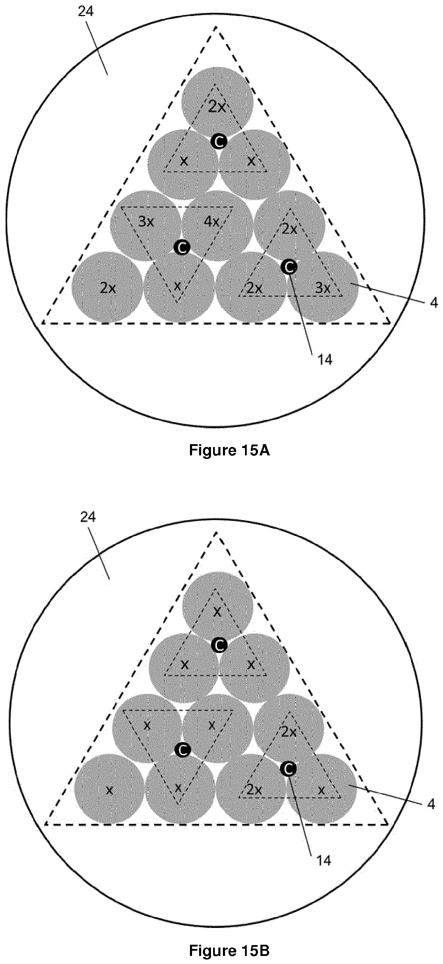

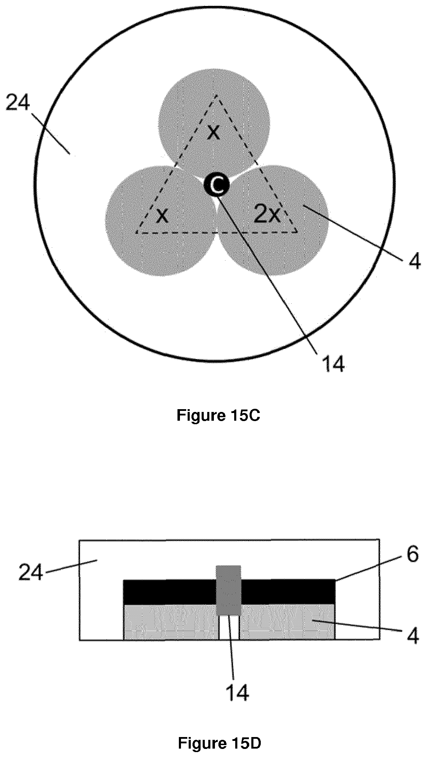

Figures 15A, 15B, 15C and 15D show diagrams of a fluidic structure according to an embodiment of an aspect of the invention. The device comprises multiple (preferably 10) triangularly arranged sweat sample areas ('cells') 4, with a central fluid channel 14 disposed between the triangularly arranged sample areas. Each central fluid channel 14 may be provided with or without a valve. A central fluid channel 14 acts as a fluid collection conduit of the three triangularly arranged sample areas 4 and the central fluid channel 14 also allows for venting of air out of the device, such that sweat can enter the device. Statistical analysis of pixelated sensor read-out (sweat volume discretization: x, 2x, 3x, etc.) is used to determine degradation and end-of-life prediction of the structure.

Figure 15A shows the discretization pattern (x, 2x, multiples of x nl sweat per sample area) at a first point in time and Figure 15B shows the discretization pattern at a second point in time occurring after the first point in time. As may be seen from comparison of the two figures, the ratios of x nl in the triangularly arranged sample areas has changed over time indicating a degradation of the device.

By monitoring the change of the discretization pattern over time, a score or probability of device degradation may be calculated and end-of life of the device predicted. For example, if the total number of even multiples of x nl exceeds 2/3rd of all sample areas, an optical, acoustic or software alarm (for example, to an application) may be generated and output to indicate to the user that the replacement and/or repair of the monitoring device is required.

Figures 15C and 15D show a base unit of triangular shaped sample collection system with integrated sensor array for discretized volume measurements and sweat gland determination. Using a triangularly arranged circular sample area (as shown in the unit cell of Figure 15C) with a central fluid channel connecting three individual sample areas (i.e., merging the discretized fluid volumes x nl, etc. from three channels into a single channel), a reduced fluid channel density can be realised enabling a device with high packing density of sample areas and more space for the sensor read-out array, such as that shown in Figure 15C. Having a reduced number of fluid channels results in an improved system architecture and form factor, and provides one more space for implementation of the sensing array to read out individual sample areas to enable the statistical analyses.

The unit cell of a triangularly shaped fluid volume-sensor system of Figures 15C and 15D comprises a fluid collection chamber 24, a central fluid channel (with or without a valve), and sampling areas 4 each with a sweat sensor disposed on top of a transparent fluid chamber.

Embodiments of aspects of the invention may also provide means for determining variation in sweat rate per gland. It is known that there is an eccrine gland size variation between body locations which also may provide a (somewhat) different sweat rate. In particular, in cases in which sweat samples are acquired from different anatomical locations (for example, through the application of multiple sweat patches), the device of Figure 11 may be utilised. With this device the ratio between single glands with respect to 'more than one gland' per sample area is statistically wise 18. This is because $P_0 + P_1$ = 0.095 and $P_1$ = 0.090 (see above). Consequently, summation of all P's with n>1 equals 1-0.095 = 0.005. Thus, $\dfrac{P_1}{P>1} = \dfrac{0.090}{0.005} = 18$

Hence the majority of the sample areas which contain glands will have single glands and hence sweat rates of individual glands can be obtained and an impression of the sweat rate variation per gland can be obtained. Even when there are two (or more) glands per sample chamber, an impression of the sweat rate variation per gland may still be obtained because there is a high possibility that the glands in this chamber are not active at the same time. As can be observed from the graph of Figure 9, the time that a gland is excreting sweat is less than the time that a gland is not excreting.

Embodiments of aspects of the invention may also provide two separate microfluidic structures in close vicinity to each other. The sweat flow rate per gland may be determined by a first microfluidic structure as denoted in the fluidic structures discussed in detail above. The first fluidic structure may be provided without a concentration sensor. A second microfluidic structure, very close to the vicinity of the first microfluidic structure, may be provided with one large sample area (for example, 1cm$^2$, constituting about 100 glands in the sample area) and a concentration sensor. The large sample area is connected to the concentration sensor by one channel, for instance, using a similar sample chamber as shown in Figure 10 but with a larger diameter. Consequently, the total sweat flow rate will be about 100 times larger and there will less delay when the sweat flows into the channel towards the concentration sensor. When it is assumed that the flow rate per gland is locally similar, the sweat flow rate per gland as determined with the first microfluidic structure is also valid for the second microfluidic structure. Moreover, in this case, the determined flow rate per gland as a function of time within the first microfluidic structure may be synchronized with the concentration determination as a function of time within the second microfluidic structure.

As may be seen from the above, embodiments of the present invention may provide means for determining an average sweat excretion rate per sweat gland of a user. Determination of the excretion rate per sweat gland allows for further analysis of the excreted sweat to be accurately performed and biomarkers in excreted sweat that vary as a function of the excretion rate per gland may be accurately assessed and analysed.

Embodiments of the present invention may be utilised in applications such as non-invasive, semi-continuous and prolonged monitoring of biomarkers that indicate health and well-being and for spot-check measurements. For example, for monitoring dehydration, stress, sleep, children's health and in perioperative monitoring. Further applications may be seen in the field of patient monitoring, for example, as early warning for sudden deterioration of hospital in-patients and for investigation of sleep disorders of out-patients, which otherwise have to be performed in spot-check fashion when a patient is visiting a doctor.

Although only a few exemplary embodiments have been described in detail above, those skilled in the art will readily appreciate that many modifications are possible in the exemplary embodiments without materially departing from the novel teachings and advantages of the embodiments of the present disclosure. The above-described embodiments of the present invention may be advantageously used independently of any other of the embodiments or in any feasible combination with one or more others of the embodiments.

Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements. In a device or apparatus claim enumerating several means, several of these means may be embodied by one and the same item of hardware. The mere fact that certain measures are recited in mutually different dependent claims does not indicate that a combination of these measures cannot be used to an advantage.

**Claims**

1. A system for determining a sweat excretion rate per sweat gland of a user, the system comprising:
   a fluidic structure configured to be in contact with the skin of the user to collect sweat excreted at the surface of the skin of the user from sweat glands, the fluidic structure comprising:

   a plurality of sample areas, each sample area comprising a cavity configured to gather excreted sweat, and two or more of the plurality of sample areas comprising a sensor configured to acquire sensor data relating to an excretion rate of the excreted sweat at the sample area; and
   a processor configured to:

   receive the sensor data from two or more of the sensors of the fluidic structure; determine, from the received sensor data, the number of sweat glands at each sample area and a total sweat excretion rate of the sample areas;
   determine a total number of sweat glands from the number of sweat glands at each sample area; and
   determine a sweat excretion rate per sweat gland using the total sweat excretion rate and the total number of sweat glands.

2. The system of claim 1, wherein
   the sensor data is an excretion rate measured at the sample area; and
   the number of sweat glands at each sample area is determined by:

   determining, from the received sensor data, a zero excretion rate that corresponds to zero sweat glands at a sample area such that sensor data below a predetermined threshold is categorised as zero excretion rate;
   determining, from the received sensor data, a base excretion rate that corresponds to one sweat gland at a sample area such that multiples of the base excretion rate correspond to multiples of one sweat gland; and
   categorising the sensor data from each sample area according to the zero excretion rate, the base excretion rate and multiples of the base excretion rate to determine the number of glands at each sample area.

3. The system of claim 2, wherein the processor is configured to:

   determine the total sweat excretion rate of the sample areas by summing the sensor data from all sample areas other than sample areas categorised as zero excretion rate;
determine the total number of sweat glands by summing the determined number of glands at each sample area; and
determine the sweat excretion rate per sweat gland by dividing the total sweat excretion rate by the total number of sweat glands.

4. The system according to any preceding claim, wherein
one or more of the plurality of cavities is segregated into a plurality of conical structures; and
the centre of each conical structure is in contact with the skin when the skin is in a first position and is not in contact with the skin when the skin is in a second position such that excreted sweat flows between the conical structures.

5. The system of any preceding claim, comprising a concentration sensor configured to determine a concentration of a compound in the excreted sweat.

6. The system according to any preceding claim, comprising three or more triangularly arranged sample areas, wherein the processor is configured to:

determine, from the received sensor data, the excretion rate at each of the three sample areas at a first time point;
determine, from the received sensor data, the excretion rate at each of the three sample areas at a second time point occurring after the first time point;
calculate a first time point ratio indicating a ratio of the excretion rates at the three sample areas at the first time point;
calculate a second time point ratio indicating a ratio of the excretion rates at the three sample areas at the second time point;
calculate a difference between the first time point ratio and the second time point ratio; and
determine, in response to the difference between the first time point ratio and the second time point ratio exceeding a predetermined threshold, degradation of the fluidic structure.

7. The system according to any preceding claim, comprising:

a supplementary fluidic structure configured to be in contact with the skin of the user to collect sweat excreted at the surface of the skin of the user from sweat glands, the supplementary fluidic structure comprising:

a supplementary sample area comprising a supplementary cavity configured to gather excreted sweat;
a supplementary channel connected to the supplementary cavity; and
a concentration sensor disposed in the supplementary channel and configured to determine a concentration of a compound in the excreted sweat, wherein

the supplementary sample area is arranged such that the excreted sweat at the supplementary sample area at least partially fills the supplementary cavity, flows into and along the supplementary channel and interacts with the concentration sensor; and
the size of the supplementary cavity of the supplementary fluidic structure is at least one hundred times larger than a cavity of the plurality of cavities of the fluidic structure.

8. The system according to any preceding claim, wherein
one or more of the plurality of sample areas further comprises a channel connected to the cavity;
the sensor is a flow sensor disposed in the channel and configured to measure an excretion rate of excreted sweat at the sample area; and
the channel and the sample area are arranged such that the excreted sweat at the sample area at least partially fills the cavity, flows into and along the channel and interacts with the flow sensor.

9. The system according to claim 8, wherein the flow sensor comprises:

an upstream temperature sensor;
a downstream temperature sensor; and
a pulsed heating element, wherein
the excretion rate is measured by:

calculating the difference between a temperature measured by the upstream temperature sensor and a temperature measured by the downstream temperature sensor with respect to the pulsed heating element to derive a flow velocity measurement; and

integrating the flow velocity measurement as a function of time to obtain the excretion rate.

10. The system according to claim 8 or 9, wherein

the fluidic structure comprises a primary intersection and an exit channel connected to the primary intersection and configured to remove the excreted sweat from the fluidic structure; and

one or more of the plurality of channels are connected to the primary intersection such that the excreted sweat flows through the channel, into the primary intersection and then into the exit channel.

11. The system according to any of claims 8 to 10, comprising:

a fluid leveller disposed in one or more of the plurality of cavities and composed of a hydrophilic material, wherein the fluid leveller is configured to direct the excreted sweat to the channel.

12. The system according to any of claims 8 to 11, comprising a de-bubbler configured to eliminate air bubbles formed in the excreted sweat, the de-bubbler comprising:

a first part comprising a plurality of hydrophilic projections arranged sequentially to define a plurality of channels between the hydrophilic projections; and

a second part comprising a hydrophobic material and a vent hole, wherein the first part is disposed opposite the second part; and

the de-bubbler is disposed in one or more of: a channel, and the exit channel.

13. The system of claim 1, wherein

the sample areas of the plurality are arranged adjacent to each other such that excreted sweat collected in a first cavity is able to overflow into an adjacent cavity;

the sensor is a colouration sensor disposed in a cavity of the plurality and configured to activate a colour change of the sample area in response to interaction with excreted sweat;

the sensor data is data indicating a colouration of a sample area; and

the processor is configured to determine the number of sweat glands by:

determining, from the received sensor data, a zero excretion rate that corresponds to zero sweat glands at a sample area at which there is no colouration;

determining, from the received sensor data, a base excretion rate that corresponds to one sweat gland at a sample area at which there is colouration at a single sample area;

determining, from the received sensor data, multiples of the base excretion rate that corresponds to multiples of one sweat gland at a sample area at which there is colouration at multiple adjacent sample areas; and

categorising the sensor data from each sample area according to the zero excretion rate, the base excretion rate and multiples of the base excretion rate to determine the number of glands at each sample area.

14. The system of claim 13, wherein the processor is configured to:

determine the sweat excretion rate at each sample area other than sample areas categorised as zero excretion rate by dividing the volume of the cavity by the time taken for the colourisation to occur at the sample area;

determine the total sweat excretion rate of the sample areas by summing the determined sweat excretion rate at all sample areas other than sample areas categorised as zero excretion rate;

determine the total number of sweat glands by summing the determined number of glands at each sample area; and

determine the sweat excretion rate per sweat gland by dividing the total sweat excretion rate by the total number of sweat glands.

15. A method for determining a sweat excretion rate per sweat gland of a user, the method comprising:

collecting, by a fluidic structure, sweat excreted at the surface of the skin of the user from sweat glands, by gathering excreted sweat at a plurality of sample areas of the fluidic structure in a cavity, each sample area comprising a cavity;

acquiring sensor data from two or more sensors at one or more of the plurality of sample areas, the sensor data relating to an excretion rate of the excreted sweat at the sample area;

receiving, by a processor, the sensor data from two or more of the sensors of the fluidic structure;

determining from the received sensor data, the number of sweat glands at each sample area and a total sweat excretion rate of the sample areas;

determining a total number of sweat glands from the number of sweat glands at each sample area; and

determining a sweat excretion rate per sweat gland using the total sweat excretion rate and the total number of sweat glands.

**Figure 1**

START

Collect, by a fluidic structure, sweat excreted at the surface of the skin of a user from sweat glands, by gathering excreted sweat at a plurality of sample areas of the fluidic structure in a cavity, each sample area comprising a cavity

S21

Acquire sensor data from two or more sensors at one or more of the plurality of sample areas, the sensor data relating to an excretion rate of the excreted sweat at the sample area

S22

Receive, by a processor, the sensor data from two or more of the sensors of the fluidic structure

S23

Determine from the received sensor data, the number of sweat glands at each sample area and a total sweat excretion rate of the sample areas

S24

Determine a total number of sweat glands from the number of sweat glands at each sample area

S25

Determine a sweat excretion rate per sweat gland using the total sweat excretion rate and the total number of sweat glands

S26

END

**Figure 2**

Figure 3

Number of glands per sample area

Figure 4

Figure 5

Figure 6

**Figure 7**

**Figure 8**

Figure 9

Figure 10

22

5

8

11

10

13

12

**Figure 11**

651

652

653

65

654

**Figure 12A**

651

655

652

Figure 12B

23

5

13

11

10

12

8

Figure 13

Figure 14A

Figure 14B

Figure 15A

Figure 15B

24

X

C

X                    2X

4

14

Figure 15C

24                                                    6

24

4

14

Figure 15D

Europäisches
Patentamt

European
Patent Office

Office européen
des brevets

# EUROPEAN SEARCH REPORT

Application Number

EP 18 20 0903

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/042585 A1 (HEIKENFELD JASON C [US]) 15 February 2018 (2018-02-15) | 1-9,15 | INV. A61B5/145 A61B5/00 |
| Y | * paragraphs [0028], [0031], [0056], [0064], [0065], [0071] - [0074] * <br> * figures 4, 9 * | 10-14 | |
| Y | US 2015/112165 A1 (HEIKENFELD JASON C [US]) 23 April 2015 (2015-04-23) <br> * paragraph [0055] * <br> * figure 6 * | 10,11 | |
| Y | EP 3 364 183 A1 (ECCRINE SYSTEMS INC [US]) 22 August 2018 (2018-08-22) <br> * paragraph [0059] * <br> * figure 8b * | 12 | |
| Y | WO 2014/143733 A1 (KENNEDY WILLIAM R [US]) 18 September 2014 (2014-09-18) <br> * paragraphs [0034], [0040], [0044], [0046], [0047] * <br> * figures 4a-c * | 13,14 | |
| A | JUNGIL CHOI ET AL: "Skin-interfaced systems for sweat collection and analytics", SCIENCE, vol. 4, no. 2, 16 February 2018 (2018-02-16), page eaar3921, XP055526287, US ISSN: 0036-8075, DOI: 10.1126/sciadv.aar3921 * the whole document * | 1-15 | TECHNICAL FIELDS SEARCHED (IPC) A61B |

The present search report has been drawn up for all claims

| Place of search | Date of completion of the search | Examiner |
|---|---|---|
| The Hague | 18 March 2019 | Trattner, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 20 0903

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2019

| Patent document cited in search report | | Publication date | Patent family member(s) | | Publication date |
|---|---|---|---|---|---|
| US 2018042585 | A1 | 15-02-2018 | EP | 3258836 A1 | 27-12-2017 |
| | | | US | 2018042585 A1 | 15-02-2018 |
| | | | WO | 2016134235 A1 | 25-08-2016 |
| US 2015112165 | A1 | 23-04-2015 | AU | 2014337151 A1 | 05-05-2016 |
| | | | CA | 2927213 A1 | 23-04-2015 |
| | | | CN | 105916433 A | 31-08-2016 |
| | | | EP | 3057491 A1 | 24-08-2016 |
| | | | JP | 2016533227 A | 27-10-2016 |
| | | | US | 2015112165 A1 | 23-04-2015 |
| | | | WO | 2015058064 A1 | 23-04-2015 |
| EP 3364183 | A1 | 22-08-2018 | CN | 108451502 A | 28-08-2018 |
| | | | EP | 3364183 A1 | 22-08-2018 |
| WO 2014143733 | A1 | 18-09-2014 | US | 2014275862 A1 | 18-09-2014 |
| | | | WO | 2014143733 A1 | 18-09-2014 |

EPO FORM P0459

For more details about this annex : see Official Journal of the European Patent Office, No. 12/82